# EUROPÄISCHE PATENTANMELDUNG

(11) **EP 4 151 275 A1**
(43) Veröffentlichungstag der Anmeldung: **22.03.2023**
(21) Anmeldenummer: 22194860.7
(22) Anmeldetag: 09.09.2022
(51) Int. Cl.: A61N 5/06, A61B 18/22, G02B 5/02, F21V 8/00

(54) **BELEUCHTUNGSSYSTEM, UMFASSEND EINEN LICHTLEITER MIT EINEM DIFFUSOR-ELEMENT**

(30) Priorität: 15.09.2021 DE 102021123831
(71) Anmelder: SCHOTT AG, 55122 Mainz (DE)
(72) Erfinder: SCHULTHEIS, Bernd, 55270 Schwabenheim (DE); WILLMES, Lothar, 65375 Oestrich-Winkel (DE); KEIPER, Oliver, 65510 Hünstetten (DE); RUSSERT, Hubertus, 55270 Jugenheim (DE); GRIMM, Jonas, 65307 Bad Schwalbach (DE); MEINL, Jürgen, 65329 Hohenstein-Holzhausen (DE); CRAMER, Martin, 65187 Wiesbaden (DE)
(74) Vertreter: Blumbach · Zinngrebe Patentanwälte PartG mbB

(57) **Zusammenfassung**

Die Erfindung betrifft ein Beleuchtungssystem insbesondere für ein medizintechnisches Therapie- und/oder Diagnosesystem, umfassend wenigstens eine Lichtquelle, vorzugsweise eine Laser-Lichtquelle, und einen Lichtleiter, der an einem proximalen Ende an die wenigstens eine Lichtquelle anschließbar und/oder dieser zuordenbar ist, und welches am distalen Ende des Lichtleiters ein Diffusor-Element mit einer Längsachse aufweist, die senkrecht zu einer Einkoppelfläche des Lichtleiters in das oder in dem Diffusor-Element verläuft, wobei das Diffusor-Element im Betriebszustand Licht über seine aktive Länge seitlich zur Längsachse abstrahlt, wobei das Diffusor-Element mindestens einen Diffusor-Grundkörper aufweist und der Diffusor-Grundkörper aus einer Matrix mit mindestens ein Streuelement beinhaltet, welche von einer festen Umhüllung zumindest an seiner Mantelfläche umschlossen ist.

Die feste Umhüllung kann einen mehrschichtigen Aufbau aus unterschiedlichen Hüllrohren aufweisen, welche unterschiedliche optische Eigenschaften aufweisen.

## Beschreibung

Die Erfindung betrifft ein Beleuchtungssystem, insbesondere für ein medizintechnisches Therapie- und/oder Diagnosesystem, sowie ein Verfahren zum Herstellen eines Diffusor-Grundkörpers mit einer festen Umhüllung, insbesondere für ein Beleuchtungssystem, und ein Verfahren zum zumindest teilweise oder abschnittsweisen Strukturieren, insbesondere zur Anpassung des Intensitätsverlaufs der seitlichen Abstrahlung, eines Diffusor-Grundkörpers. Das Beleuchtungssystem umfasst dabei einen Lichtleiter und ein Diffusor-Element mit einer festen Umhüllung.

Derartige Beleuchtungssysteme kommen verstärkt im medizinischen Umfeld zum Einsatz. Derzeit lassen sich folgende Anwendungsschwerpunkte klassifizieren:
- Photodynamische Therapie (PDT) oder Photo-Immuno-Therapie (PIT) zur Tumortherapie
- Endovenöse Lasertherapie (EVLT) zur Behandlung von Krampfadern
- Laser induzierte interstitielle Thermotherapie (LITT) sowie
- sonstige Anwendungen, u.a. im Bereich der Dentalmedizin, Augenheilkunde sowie Dermatologie.

Die Photodynamische Therapie (PDT) ist eine minimalinvasive Therapiemöglichkeit bei verschiedenen Krebserkrankungen. Unter der PDT versteht man ein Verfahren zur Behandlung von Tumoren und anderen Gewebeveränderungen (wie beispielsweise Gefäßneubildungen) mit Licht in Kombination mit einer lichtaktivierbaren Substanz. Zu Beginn der Behandlung werden den Patienten intravenös lichtsensible Substanzen, sogenannte Photosensitizer in die Blutbahn injiziert, die sich in beziehungsweise an den Krebszellen anreichern. Diese natürlichen Photosubstanzen konzentrieren sich in den Tumorzellen und bewirken dort eine starke Lichtempfindlichkeit. Dazu werden während der PDT-Behandlung in das Tumor-Gewebe mehrere Kanülen (typ. bis zu 8) gestochen, in die jeweils ein Lichtleiter mit einem Diffusor-Element eingeführt wird, wobei die Diffusor-Elemente möglichst räumlich über das Tumor-Gewebe verteilt angeordnet sein müssen. Laser-Licht, in der Regel mit Wellenlängen im sichtbaren Spektralbereich, zum Beispiel Grün-Licht mit 532 nm oder Rot-Licht mit 690 nm Wellenlänge, wird über die Lichtleiter in die Diffusor-Elemente eingekoppelt, so dass das Tumor-Gewebe möglichst gleichmäßig von innen ausgeleuchtet wird. Dabei bilden sich in diesen Zellen aggressive Sauerstoffradikale, welche die Tumorzellen selektiv zerstören. Im Gegensatz zu den kranken Zellen bleiben die gesunden Zellen von dieser chemischen Reaktion unberührt. Der genaue Wirkmechanismus ist u.a. in "Photodynamic Therapy of Cancer", Cancer Medicine, 2003 beschrieben.

Bei der Photo-Immuno-Therapie (PIT) wird mit entsprechend modifiziertem Photosensitizer dagegen eine Immun-Reaktion an bzw. in der Krebszelle ausgelöst, die bei Lichtbestrahlung zum Absterben der Krebszelle führt.

Man unterscheidet hier zwischen Zylinder-Diffusoren mit typischen aktiven Längen von 10 bis 50 mm, Spot-Diffusoren, die einen vorwärts gerichteten Beleuchtungskegel erzeugen sowie Punktstrahler, die eine radiale Lichtemission aufweisen.

Bei den Zylinder-Diffusoren kommt es im Betriebszustand insbesondere auf eine möglichst homogene seitliche Abstrahlung der Diffusor-Elemente über deren Länge an. Dies sowohl axial, d.h. an allen Punkten entlang jeder Linie vom proximalen zum distalen Ende in Richtung der Längsachse ist die Abstrahlungsintensität im Rahmen der Homogenitätsanforderung gleich, als auch radial, d.h. an allen Punkten jeder Umfangslinie entlang der Längsachse ist die Abstrahlungsintensität im Rahmen der Homogenitätsanforderung ebenfalls gleich, womit diese Diffusoren nahezu als Lambertsche Strahler wirken.

Gleichzeitig muss auch eine hohe Streueffizienz erzielt werden, um möglichst einen geringen Wärmeeintrag ins Gewebe sicher zu stellen. Typische Homogenitätsanforderungen an die seitliche Abstrahlung liegen bei maximal ± 10 bis 20 % Abweichung von der mittleren Intensität, wobei eine vorwärts gerichtete Abstrahlung, insbesondere aus dem distalen Ende heraus, von mehr als 10 % des eingekoppelten Lichtes, typischerweise max. 5 %, zu vermeiden ist. Die typische Laser-Leistung liegt bei den PDT-Anwendungen bei < 5 W Dauerleistung, so dass pro cm Diffusorlänge max. zwischen 100 mW und 1000 mW, typischerweise zwischen 200 mW und 500 mW, abgestrahlt werden. Dies erlaubt derzeit den Einsatz von Kunststoffbasierten Diffusor-Ansätzen.

Die Schriften EP 2062077 A4, US 2009/0204111 A1 und DE 102015119875 A1 beschreiben Diffusoren mit einer Faser.

Die Schrift EP 2062077 A4 beziehungsweise WO 2008/024397 A2 beschreibt u.a. einen Diffusor zum Ausgeben von optischer Energie hoher Leistungsdichte zu einer Behandlungsstelle am distalen Ende mindestens einer optischen Faser. Vorgeschlagen wird ein Diffusor umfassend einen Abschnitt einer vorbestimmten Länge am distalen Ende einer optischen Faser sowie Streuungszentren, die in dem Abschnitt der vorbestimmten Länge positioniert sind, wobei die Streuungszentren bewirken, dass ein Teil der eingegebenen optischen Energie radial auf eine Behandlungsstelle austritt. Die Streuungszentren können Streupartikel sein, die in dem Kern oder der Umhüllung des Kerns enthalten sind. Neben aufwendiger und schwer kontrollierbarer Einbringung bzgl. beispielsweise der Verteilung und/oder Größe der oben genannten Nanocracks oder Nano-Hohlräume, können diese sich auch negativ auf die Bruchanfälligkeit des Bauteiles auswirken. Des Weiteren ist damit zu rechnen, dass aufgrund des exponentiellen Abfalls der seitlichen Emission oder ungleichmäßiger Verteilungen eine seitliche Abstrahlung nicht mit geforderter Homogenität erzielt werden kann.

Vorteile würde hier ein Zylinder-Diffusor mit sich bringen, wie er bei PDT-Anwendungen zum Einsatz kommt. Allerdings sind bei der EVLT-Behandlung deutlich höhere Laser-Leistungen erforderlich. So beträgt die Laserleistung typischerweise zwischen 10 und 50 W bei Wellenlängen im NIR-Bereich, d.h. zwischen etwa 800 nm und 1480 nm, welche derzeit mit Dioden-Lasern (zum Beispiel 810 nm, 940 nm oder 1480 nm) oder Nd: YAG-Lasern (1064 nm) bereitgestellt wird.

Inzwischen haben sich auch größere Wellenlängen um 2 µm zur EVLT-Behandlung etabliert. Zum Einsatz kommen dann beispielsweise Tm: YAG-Laser (1,9 µm) und Ho:YAG-Laser (2,1 µm). Aufgrund der Absorptionseigenschaften von Gewebe werden bei diesen Wellenlängen geringere Laserleistungen, typischerweise < 10 W, benötigt. Allerdings kommen hier bereits zwingend Quarzglas-Lichtleiter insbesondere für die Zuführung des Laserlichtes zum Einsatz.

Die Homogenitätsanforderungen an die seitliche Abstrahlung von Diffusoren, welche für EVLT eingesetzt werden können, sind gegenüber einer PDT-Anwendung weniger hoch und können bei maximal ± 30 % bis maximal ± 50 % Abweichung von der mittleren Intensität betragen.

Bei der LITT handelt es sich um ein minimal-invasives Verfahren, das zur lokalen Tumordestruktion eingesetzt wird. Dabei wird unter bildgebender Kontrolle (zum Beispiel Sonographie/ MRT) der Tumor punktiert, eine (oder mehrere) Laserfaser(n) in den Tumorherd eingebracht und dieser durch thermische Energie verödet. Zum Einsatz kommen hier insbesondere Nd:YAG-Lasern (1064 nm), Halbleiter-Laser (980 nm) sowie Diffusor-Tip-Applikatoren. Die Laserleistung liegt bei etwa 5 bis 8 W (s. u. a. "Laserinduzierte Interstitielle Thermotherapie (LITT) bei malignen Tumoren", BÄK und KBV 01/2002).

In der Schrift WO 2019/063799 A1 der Anmelderin wird ein Beleuchtungssystem mit einem Lichtleiter und einem Diffusor-Element sowie ein Verfahren zum Herstellen und/oder zum zumindest teilweise oder abschnittsweisen Strukturieren eines Diffusor-Grundkörpers beschrieben, welches bereits hohe Homogenitätsanforderungen an die seitliche Abstrahlung von Diffusoren ermöglicht.

Der beschriebene Diffusor-Grundkörper umfasst einen Lichtleiter sowie Einrichtungen zur Homogenisierung der Abstrahlungsintensität entlang der Längsachse des Diffusor-Grundkörpers. Es kann eine Intensitätsverteilung der seitlichen Abstrahlung erreicht werden, welche um höchstens ± 50 %, bevorzugt höchstens ± 30 % und meist bevorzugt höchstens als ± 5 % von der mittleren seitlichen Abstrahlungsintensität abweicht.

Wie Messungen an einer Vielzahl derart aufgebauter bzw. derart hergestellter Zylinder-Diffusoren gezeigt haben, können damit bereits gute Homogenitätswerte in der Abstrahlcharakteristik sowie hohe Effizienzwerte erzielt werden, wie sie für die eingangs beschriebenen Anwendungen erforderlich sind.

Allerdings zeigt sich auch, dass es insbesondere bei bestimmten Leistungswerten der Laserstrahlung bzw. des Lichts zu Rückstreueffekten kommen kann.

So kann etwa Licht in den Lichtleiter hinein zurückgestrahlt werden, wobei es sogar bis zur Laser-Lichtquelle gelangen kann. Dabei kann es zu unerwünschten Leuchterscheinungen in dem Lichtleiter kommen. Zudem kann die Laser-Lichtquelle je nach Höhe der Intensität des rückgestreuten Lichtes instabil werden, was dazu führen kann, dass sie dann ggf. abschaltet.

Weiterhin kann es infolge der Rückreflektion lokal zu Hotspots kommen, bei denen eine unerwünschte Temperaturerhöhung des Beleuchtungssystems auftreten kann. Dies ist bei bestimmten Anwendungsfällen unerwünscht und kann eine Behandlung negativ beeinflussen.

Es ist daher Aufgabe der Erfindung, die zuvor genannten Nachteile zu minimieren und dabei die hohe Homogenität und Effizienz weiter zu steigern bzw. zumindest zu erhalten.

### Offenbarung der Erfindung:

Die Aufgabe der Erfindung wird bereits durch den Gegenstand der unabhängigen Ansprüche gelöst, wobei vorteilhafte Weiterentwicklungen den abhängigen Ansprüchen sowie der weiteren Offenbarung der Beschreibung und der Zeichnungen zu entnehmen sind.

Hierzu wird ein Beleuchtungssystem, insbesondere für ein medizintechnisches Therapie- und/oder Diagnosesystem vorgeschlagen,
umfassend wenigstens eine Lichtquelle, insbesondere eine Laser-Lichtquelle, und einen Lichtleiter, der an einem proximalen Ende an die wenigstens eine Lichtquelle anschließbar und/oder angeschlossen ist,
und welches am distalen Ende des Lichtleiters ein Diffusor-Element mit einer Längsachse aufweist, die senkrecht zu einer Einkoppelfläche des Lichtleiters in das Diffusor-Element verläuft,
wobei das Diffusor-Element im Betriebszustand Licht über seine aktive Länge seitlich zur Längsachse abstrahlt,
wobei das Diffusor-Element mindestens einen Diffusor-Grundkörper aufweist, welcher eine Matrix mit mindestens einem Streuelement umfasst, welche von einer festen Umhüllung zumindest abschnittsweise an seiner Mantelfläche umschlossen ist, und
wobei die feste Umhüllung mit einem mehrteiligen oder mehrschichtigen Aufbau ausgebildet ist, umfassend zumindest zwei Hüllrohre oder Schichten, bevorzugt zumindest drei Hüllrohre oder Schichten.

Die Lichtquelle kann eine Laser-Lichtquelle oder halbleiterbasierte Lichtquelle oder lichtemittierende Diode (LED), insbesondere auch eine Laserdiode (LD), oder einen Laser umfassen.

Der Diffusor-Grundkörper ist demnach in einer bevorzugten Ausführungsform der Erfindung zumindest teil- oder abschnittsweise oder bevorzugt auch vollständig an seiner Mantelfläche von einer festen Umhüllung umschlossen bzw. umschliessbar, wobei diese feste Umhüllung in einer bevorzugten Ausführungsform der Erfindung eine Abfolge von Hüllrohren sein kann. Die Hüllrohre können unterschiedliche optische Eigenschaften aufweisen.

Die feste Umhüllung kann aber auch einen mehrschichtigen oder mehrkomponentigen bzw. mehrteiligen Aufbau umfassen, also zumindest eine Schicht, welche auf einem Hüllrohr oder zwischen Hüllrohren aufgebracht sein kann. Demnach kann eine feste Umhüllung zwei Hüllrohre umfassen. Zusätzlich kann die feste Umhüllung eine Schicht und/oder ein weiteres Hüllrohr umfassen oder eine andere Kombination von Hüllrohren und/oder Schichten, insbesondere zumindest drei, bevorzugt drei aufeinanderfolgende Hüllrohre.

Das erfindungsgemäße Beleuchtungssystem kann ferner durch zumindest eines der folgenden Merkmale gekennzeichnet sein:
das zumindest eine Streuelement ist entlang der Längsachse des Diffusor-Grundkörpers im Wesentlichen zu dieser parallel ausgerichtet oder in einem Winkel zur Längsachse angeordnet,
am distalen Ende des Diffusor-Grundkörpers und/oder dem Übergangsbereich zwischen Lichtleiter und Diffusor-Grundkörper und/oder dem Diffusor-Grundkörper sind, diesen zumindest teilweise oder abschnittsweise umschließend, Einrichtungen zur Homogenisierung der Abstrahlungsintensität entlang der Längsachse des Diffusor-Grundkörpers vorgesehen,
der Diffusor-Grundkörper weist an seinem distalen Ende eine Reflektorfläche auf, mit der das Diffusor-Grundkörper durchlaufende Licht im Betrieb zumindest teilweise wieder zurück reflektierbar ist, und/oder
das Beleuchtungssystem weist im Betriebszustand eine Intensitätsverteilung der seitlichen Abstrahlung auf, die um höchstens ± 50 %, bevorzugt höchstens ± 30 % und meist bevorzugt höchstens als ± 5 % von der mittleren seitlichen Abstrahlungsintensität abweicht.

Die mittlere seitliche Abstrahlungsintensität meint dabei den Mittelwert der seitlichen Abstrahlungsintensität über die Länge des Diffusor-Grundkörpers gemessen.

Als seitliche Abstrahlung wird im Rahmen der vorliegenden Offenbarung eine Abstrahlung verstanden, welche Richtungsanteile aufweist, die in radialer Richtung ausgehend von der Längsachse des Diffusor-Grundkörpers verlaufen. Als seitliche Abstrahlungsintensität wird die Intensität dieser Abstrahlung verstanden.

Als zurück reflektiertes Licht wird eine Strahlung verstanden, die von der Reflektorfläche am distalen Ende des Diffusor-Grundkörpers in diesen zurückreflektiert wird, wobei dabei auch Licht über die feste Umhüllung oder das Hüllrohr geführt werden kann.

Das zumindest eine Streuelement kann entlang der kompletten Längsachse des Diffusor-Grundkörpers mit einheitlichem Querschnitt im Wesentlichen zu dieser parallel oder bei sich verjüngenden Diffusor-Grundkörpern in einem Winkel zur Längsachse angeordnet sein. Das zumindest eine Streuelement kann vorteilhaft auch rohrförmig und insbesondere koaxial zur Längsachse angeordnet sein.

Denkbar sind auch Streubereiche, welche um die Längsachse des Diffusorgrundkörpers, insbesondere entlang dieser, angeordnet sind. Gemäß einer Ausführungsform winden sich der Streubereich bzw. die Streubereiche spiralförmig um die Längsachse des Diffusor-Grundkörpers. Als besonders vorteilhaft haben sich Ausführungsformen herausgestellt, bei denen das Streuelement oder der Streubereich mit einer konstanten Steigung um die Längsachse des Diffusor-Grundkörpers angeordnet sind. Hierbei kann der Streubereich in Form einer Helix vorliegen, die sich um die Längsachse des Diffusor-Grundkörpers windet.

Die einzelnen Streubereiche können durch ein oder mehrere Streuelemente gebildet werden. Eine Ausführungsform sieht vor, dass der Streubereich durch ein Streuelement in Form einer Helix oder Spirale gebildet wird. Derartige Streuelement- bzw. Streubereichsanordnungen im Grundkörper haben den Vorteil, dass damit eine erhöhte Streuwechselwirkung mit Achs-parallelen Lichtanteilen bzw. für Lichtanteile, welche unter kleinem Winkel zur Diffusor-Achse verlaufen, erfolgen und somit die Streu-Effizienz erhöht werden kann. Zudem kann damit die Homogenität der Abstrahlung optimiert werden.

Eine Mehrzahl von Streuelementen kann in einer bestimmten vorgebbaren geometrischen Anordnung um die Längsachse des Diffusor-Grundkörpers angeordnet sein, bevorzugt in einer regelmäßigen Struktur um diese, insbesondere bevorzugt kreisförmig. Eine Mehrzahl von unter einem Winkel angeordneten Streuelementen trifft sich somit bevorzugt in einem Fluchtpunkt außerhalb des Diffusor-Grundkörpers.

Am distalen Ende des Diffusor-Grundkörpers, dem Übergangsbereich zwischen Lichtleiter und Diffusor-Grundkörper sind bevorzugt Einrichtungen und/oder Maßnahmen zur Homogenisierung der seitlichen Abstrahlung entlang der Längsachse vorgesehen, die den Diffusor-Grundkörper zumindest teil- oder abschnittsweise und/ oder im Wesentlichen komplett umschließen.

Beispielsweise gehören zu den Einrichtungen Hülsen, Hüllen, Kappen und/oder Schichten am distalen Ende des Diffusors, um eine vorwärts gerichtete Abstrahlung aus dem distalen Ende zu verhindern beziehungsweise diese zurück zu reflektieren und damit den Streuprozessen im Diffusor-Grundkörper erneut bereitzustellen und andererseits Streulichteffekte und/oder Lichtreflexe am distalen Ende des Diffusor-Grundkörpers zu vermeiden.

Ähnliches gilt für den Übergangsbereich zwischen Lichtleiter und Diffusor-Grundkörper. Auch hier können Streulichteffekte und/oder Lichtreflexe auftreten, die durch entsprechend wirkende Elemente, beispielsweise Hülsen und/oder Schichten an dieser Stelle reduziert werden können.

Der Lichtleiter kann eine Einzelfaser, beispielsweise Mono- oder Multimodelichtleitfaser, aufweisend einen Kern mit einem Kerndurchmesser und einen Mantel oder ein Faserbündel mit einem Faserbündeldurchmesser, umfassen.

Damit lassen sich reproduzierbar und auch kostenoptimiert, im Betriebszustand homogen abstrahlende Diffusor-Elemente für medizinische Therapien, wie sie eingangs erwähnt sind, bereitstellen.

In einer bevorzugten Ausführungsvariante ist vorgesehen, dass die Streuelemente im Diffusor-Grundkörper radial gleichmäßig verteilt um die Längsachse des Diffusor-Grundkörpers angeordnet sind, wobei eine Kernzone um die Längsachse keine oder eine deutlich reduzierte Anzahl von Streuelementen je Flächeneinheit gegenüber der Anzahl von Streuelementen je Flächeneinheit außerhalb der Kernzone aufweist und somit die Streuelemente überwiegend außerhalb dieser Kernzone in der Matrix angeordnet sind.

Damit kann erreicht werden, dass das eingekoppelte Licht, welches in der Regel mit geringer NA (< 0,3, typischerweise um die 0,2) eingekoppelt wird, nicht sofort an den Streuelementen gestreut wird. Andererseits kann durch die nahezu Streuelement-freie Kernzone genügend Licht ohne Streuung bis zum distalen Ende des Diffusor-Grundkörpers geleitet werden. Damit kann einerseits die Intensität nahe der Einkoppelstelle (proximales Ende des Diffusor-Grundkörpers) reduziert und andererseits die Intensität nahe dem distalen Ende Diffusor-Grundkörpers erhöht werden.

In einer weiteren bevorzugten Ausführungsvariante kann vorgesehen sein, dass der Diffusor-Grundkörper bezogen auf seine Querschnittsfläche eine Matrix aufweist, welche unterschiedliche Brechungsindices n₁ und n₁', insbesondere zwischen der Kernzone und dem Randbereich der Matrix, aufweist, in die die Streuelemente eingelagert sind. Damit kann bspw. die numerische Apertur NA in der Kernzone mit einem Matrix-Brechungsindex n₁ und außerhalb der Kernzone der Matrix mit einem Brechungsindex n₁' beeinflusst werden.

Weiterhin kann damit die Ausbreitung des Lichtes im Diffusor-Grundkörper und somit eine Anregung der Streuzentren über die Länge des Diffusors an die erforderliche Abstrahlcharakteristik angepasst werden. Zudem kann beim Herstellprozess eine beliebige Querschnittsgeometrie der Kernzone mit dem Brechungsindex n₁, d.h. z.B. von im Wesentlichen kreisrunden bis hin zu einer polygonen oder sternförmigen Form realisiert werden.

Unterstützt werden kann die Homogenisierung der Intensität der seitlichen Abstrahlung, wenn der Durchmesser des Diffusor-Grundkörpers, in den die Streuelemente eingebettet sind, gleich groß oder größer ist als ein Kerndurchmesser oder Faserbündeldurchmesser des Lichtleiters.

Als besonders günstig hat sich ein Verhältnis zwischen Kerndurchmesser bzw. Faserbündeldurchmesser des Lichtleiters und Durchmesser der Matrix von ≤ 1,0 bis 0,7, besonders bevorzugt von ≤ 1,0 bis 0,8 herausgestellt.

Ein nur geringfügig gegenüber dem Durchmesser der Matrix kleinerer Kerndurchmesser bzw. Faserbündeldurchmesser kann dabei einen Intensitäts-Peak an der Einkoppelstelle (Übergangsbereich von Lichtleiter und Diffusor-Grundkörper) reduzieren.

Ein deutlich kleinerer Kerndurchmesser bzw. Faserbündeldurchmesser gegenüber dem Durchmesser der Matrix des Diffusor-Grundkörpers, das heißt ein Verhältnis von < 0,8, kann dagegen zu einer Intensitätsabsenkung an der Einkoppelstelle führen, die für bestimmte Anforderungen ebenfalls von Vorteil sein kann.

Liegt das Verhältnis zwischen 1 und 0,9, hat sich zudem herausgestellt, dass eine besonders robuste mechanische Kopplung bzw. Verbindung, zum Beispiel mittels Spleißen, zwischen Lichtleiter und Diffusor-Grundkörper erzielt werden kann.

In bevorzugter Ausführungsform weist das Diffusor-Element zwischen dem proximalen Ende des Diffusor-Grundkörper und dem distalen Ende des Lichtleiters, eine Verbindungszone auf, die form- und/oder stoffschlüssig mittels Verkleben, Spleißen oder Verpressen hergestellt ist und die zumindest den Durchmesser des Diffusor-Grundkörpers und den Kerndurchmesser oder den Faserbündeldurchmesser des Lichtleiters verbindet.

Zum Anpassen gegebenenfalls unterschiedlicher thermischer Ausdehnungskoeffizienten kann es von Vorteil sein, wenn zwischen dem proximalen Ende des Diffusor-Grundkörper und dem distalen Ende des Lichtleiters zusätzlich in der Verbindungszone ein Zwischenmedium vorgesehen ist. Dieses kann beispielsweise ein Übergangsglas oder Lotglas sein. Andererseits kann dies auch ein transparenter dauerelastischer Kleber sein. Des Weiteren kann in der Verbindungszone ein optisches Element angeordnet sein oder die Verbindungszone als optisches Element ausgeführt sein, um beispielsweise die Strahlführung und/oder Lichtlenkung durch geometrische oder Anpassung von Brechwerten zu modifizieren.

Der Diffusor-Grundkörper kann im Wesentlichen aus einer Matrix aus transparentem Kunststoff, Glas, Quarzglas oder Glaskeramik bestehen, wobei die darin eingelagerten Streuelemente beispielsweise bei einer Kunststoff-Matrix aus einem porösem oder pigmentierten oder zum Beispiel weiß eingefärbten Kunststoff, bei einer Glas-Matrix aus Poren, Partikeln, porösem oder pigmentierten oder zum Beispiel weiß eingefärbten oder Inhomogenitäten beinhaltendem Glas- oder Glaskeramik-Elementen und den darin umfassten Kristalliten, bei einer Quarz-Matrix aus Poren, porösem Quarzglas oder keramischen beziehungsweise polykristallinen Partikeln oder bei einer transparenten Glaskeramik-Matrixaus Poren, Partikeln, porösem oder pigmentierten oder zum Beispiel weiß eingefärbten oder Inhomogenitäten beinhaltendem Glas- oder Glaskeramik-Elementen und den darin umfassten Kristalliten bestehen können.

Dabei können vorteilhaft auch Kombinationen der beispielhaft genannten Streuelemente in der jeweiligen Matrix vorliegen. Die Inhomogenitäten des Glases oder der Glaskeramik, die die Streuelemente bei Glas- oder Glaskeramikmatrixlösungen bilden können, umfassen beispielsweise Phasenseparationen, Entmischungen und/oder partikuläre Einlagerungen, Keime und/oder Kristallite.

Hierbei soll die Konzentrationen der Streuelemente im Streubereich von 10 ppm bis 1000 ppm und bevorzugt von 20 ppm bis 100 ppm betragen. Hierbei bezieht sich die Konzentrationsangabe in ppm auf den Anteil der Streupartikel im Verhältnis zu den Masseanteilen der Bestandteile des jeweiligen Materials, insbesondere den Kunststoff, die Glas-Matrix oder die Quarz-Matrix, in welchem die Streupartikel eingelagert sind. Hierbei weisen die jeweils gebildeten Streuelemente, diese bedeutet beispielsweise die Poren, Partikel, porösen oder pigmentierte oder zum Beispiel weiß eingefärbten oder Inhomogenitäten beinhaltenden Glas- oder Glaskeramik-Elemente und die darin umfassten Kristallite, bevorzugt einen Durchmesser von 10 nm bis 1000 nm auf, besonders bevorzugt von 100 nm bis 800 nm auf.

Ein Kunststoff-basierter Lösungsansatz für den Diffusor-Grundkörper aus Kunststoffstäben aus zum Beispiel PMMA, PET oder PC lässt sich bereits bei niedrigen Prozesstemperaturen bei dessen Herstellung oder Umformung realisieren. Allerdings weisen derart aufgebaute Diffusor-Grundkörper entsprechend eine eher niedrige thermische Beständigkeit auf, und sind daher eher für Anwendungen mit niedriger Laserleistung geeignet. Zudem eigenen sich diese nur für Anwendungen im sichtbaren Spektralbereich (VIS), da Kunststoffe eine in der Regel hohe Absorption im NIRbeziehungsweise IR-Bereich aufweisen.

Glas-basierte Ansätze sind hier wesentlich robuster und vor allem thermisch stabiler, so dass auch größere Laserleistungen applizierbar sind. Als Elemente zum Aufbau des Diffusor-Grundkörpers kommen beispielsweise Stäbe der Gläser vom Typ N-BK7, optisches Bor-Kronglas der Anmelderin, Borosilikatglas oder Pb- beziehungsweise Schwermetall-freie in Betracht, wie sie unter anderem als Kernglas für optisch hochwertige Glasfasern für beispielsweise Endoskope oder Dentalstäbe zur Aushärtung von Zahnfüllungen zum Einsatz kommen. Mit letzteren können zukünftige RoHS-Anforderungen erfüllt werden. Derartige Gläser sind in den Druckschriften DE 10 2012 100 233 A1 sowie in der DE 10 2013 208 838 B4 der Anmelderin beschrieben, welche hiermit vollumfänglich inkorporiert werden.

Beispiele für solche Gläser für die Lichtleitstäbe beziehungsweise für die Matrix des Diffusor-Grundkörpers aus dem Bereich der bleifreien Zinn-Silikat-Gläser beziehungsweise Alkali-Zink-Silikat Gläser beinhalten folgende Komponenten (angegeben in Gew.-% auf Oxidbasis):

| | von | bis |
|---|---|---|
| B₂O₃ | 0 | 24 |
| SiO₂ | 23 | 62,1 |
| Al₂O₃ | 0 | 10 |
| Li₂O | 0 | 10 |
| Na₂O | 0 | 18,5 |
| K₂O | 0 | 25,7 |
| BaO | 0 | 57,8 |
| ZnO | 0 | 40 |
| La₂O₃ | 0 | 25 |
| ZrO₂ | 0 | 10 |
| HfO₂ | 0 | 14,2 |
| SnO₂ | >0 | 2 |
| MgO | 0 | 8 |
| CaO | 0 | 8 |
| SrO | 0 | 24,4 |
| Ta₂O₅ | 0 | 22 |
| Y₂O₃ | 0 | 11,9 |
| Rb₂O | 0 | 15 |
| Cs₂O | 0 | 21 |
| GeO₂ | 0 | 7,5 |
| F | 0 | 2 |
| Σ R₂O | 5 | 20 |
| Σ MgO, CaO, SrO, ZnO | 20 | 42 |

Die Hüllrohre sind beispielsweise aus einer der folgenden Gruppe 1 bis 4 ausgewählt, welche jeweils nachfolgende Komponenten beinhalten (angegeben in Gew.-% auf Oxidbasis):

| | **Gruppe 1** | **Gruppe 2** | **Gruppe 3** | **Gruppe 4** |
|---|---|---|---|---|
| SiO₂ | 70 - 78 | 63 - 75 | 75 - 85 | 62 - 70 |
| Al₂O₃ | 5-10 | 1 -7 | 1 -5 | 1 -10 |
| B₂O₃ | 5-14 | 0-3 | 10-14 | > 15 |
| Li₂O | 0-2 | 0-1 | 0-3 | 0-2 |
| Na₂O | 0-10 | 8 - 20 | 2-8 | 0-10 |
| K₂O | 0-10 | 0-6 | 0-1 | 0-10 |
| MgO | 0-1 | 0-5 | 0 | 0-5 |
| CaO | 0-2 | 1 -9 | 0 | 0-5 |
| SrO | 0-1 | 0 | 0 | 0-5 |
| BaO | 0-4 | 0-5 | 0 | 0-5 |
| F | 0-1 | 0-1 | 0 | 0-1 |
| Cl | 0-1 | 0-1 | 0 | 0-1 |
| Fe₂O₃ | 0-2 | 0-2 | 0-2 | 0-2 |

Weist die feste Umhüllung nun einen mehrschichtigen Aufbau auf oder insbesondere zumindest zwei Hüllrohre auf, kann insbesondere damit erreicht werden, dass gestreute, geführte sowie reflektierte oder zurück reflektierte Lichtanteile definiert ebenfalls zur seitlichen Abstrahlung entlang der Diffusorlänge beitragen.

Besonders erfolgreich gelingt dies bereits bei einem Aufbau mit mindestens zwei Hüllrohren, wobei das innere, erste Hüllrohr vorzugsweise aus einem transparenten Borosilikatglas und das sich daran anschließende zweite Hüllrohr aus einem transluzenten, streuenden Glas aufgebaut ist, in welches Streuzentren eingelagert sein können. Diese Streuzentren können Partikel, Phasengrenzen oder Entmischungen sein.

Das zweite Hüllrohr kann in einer besonders bevorzugten Ausführungsform ein Weißglas umfassen. Ein Weißglas kann weiße Pigmente umfassen, um einen weißlichen Farbeindruck zu bewirken.

Ein Weißglas-Hüllrohr kann gemäß einer bevorzugten Ausführungsform ein, vorzugsweise transluzentes, Silikat-Weißglas sein oder umfassen. Dieses kann eine extreme Streuwirkung aufweisen. Es kann beispielsweise ein As-Pbhaltiges Silikatglas sein. Ein solches Glas ist ein Silikatglas, welches Blei (Pb) und Arsen (As) beinhaltet. Zur Streuung können inhomogene Bereiche in dem Glas als Streuelement dienen, welche einen gegenüber dem umgebenden Glas erhöhten Gehalt an Blei und/oder Arsen aufweisen können. Alternativ können selbstverständlich auch Streuelemente, etwa Streupartikel, eingelagert sein und die Streuzentren ausbilden.

In einer weiteren, ebenfalls bevorzugten Ausführungsform der Erfindung ist ein weiteres, drittes Hüllrohr für die feste Umhüllung vorgesehen. Dieses dritte Hüllrohr kann das äußere Hüllrohr der festen Umhüllung darstellen. Das erste und das zweite Hüllrohr können dabei wie vorstehend ausgeführt ausgebildet sein.

Gemäß einer Ausführungsform kann anstatt eines zweiten Hüllrohres aus einem Glas mit hoher Streuzentrendichte, beispielsweise aus Weißglas, auch ein Ring aus einzelnen Glasstäbchen mit hoher Streuzentrendichte bzw. aus einzelnen Weißglasstäbchen verwendet werden. Die ringförmig angeordneten Glasstäbchen werden hierbei zwischen dem ersten innen liegenden Hüllrohr und dem dritten außenliegenden Hüllrohr platziert. Beim Ziehprozess verschmelzen die Glasstäbchen miteinander und bilden eine homogene streuende Schicht. Durch die ringförmige Anordnung der Glasstäbchen ist diese im Wesentlichen ring- bzw. rohrförmig ausgebildet

Mit diesen Ausbildungen der festen Umhüllung kann höchst vorteilhaft erreicht werden, dass nur noch ein sehr geringer Anteil über die Koppelstelle zum Lichtleiter oder in diesen Lichtleiter hinein oder sogar weiter in Richtung der Laser-Lichtquelle zurückgeführt wird. Auf diese Weise kann höchst vorteilhaft sichergestellt werden, dass kein oder nur ein geringer Anteil des Lichtes, welcher vorzugsweise weniger als 10 % des abgegebenen Lichtes der Laser-Lichtquelle beträgt, noch weiter bevorzugt weniger als 5 % des abgegebenen Lichtes, wieder in den zuführenden Lichtleiter zurückgestrahlt wird bzw. in diesen eintritt.

Es hat sich als günstig herausgestellt, wenn sich zumindest zwei der Hüllrohre der festen Umhüllung in zumindest einer optischen Eigenschaft voneinander unterscheiden. Die optische Eigenschaft meint in diesem Zusammenhang beispielsweise Eigenschaften hinsichtlich der Transparenz bzw. Transluzenz, wobei ein entsprechender Unterschied beispielsweise bei einem klar transparenten Hüllrohr und einem transluzenten Hüllrohr gegeben ist, des Brechungsindex und/oder des Materials des Hüllrohrs.

Ergänzend oder alternativ kann ein Unterschied in einer optischen Eigenschaft auch ein unterschiedliches Transmissionsverhalten der entsprechenden Hüllrohre meinen, etwa das Transmissionsverhalten von elektromagnetischer Strahlung, vorzugsweise im Spektrum der Anwendungswellenlängen.

Ergänzend oder alternativ kann ein Unterschied in einer optischen Eigenschaft auch einen Unterschied im Wert des Brechungsindex der entsprechenden Hüllrohre meinen, etwa einen Unterschied im Wert des Brechungsindex von beispielsweise etwa 0.05 oder etwa 0.1.

Eine feste Umhüllung, umfassend beispielsweise zumindest drei Hüllrohre mit unterschiedlichen optischen Eigenschaften, kann die Gefahr von unerwünschten Leuchterscheinungen in oder an dem Lichtleiter, insbesondere an auch dessen Enden bzw. Übergang zum Diffusorkörper nochmals signifikant verringern. Zudem kann so auch verhindert werden, dass die Lichtquelle instabil wird oder sich sogar abschaltet, so dass eine größere Sicherheit in der Behandlung gegeben ist. Dies trifft insbesondere auf Laser-Lichtquellen zu.

Auch können besonders vorteilhaft lokale Hotspots oder andere Leuchterscheinungen vermieden werden, welche etwa an der Spleißstelle, also dem Übergang von Lichtleiter zu Diffusorkörper, infolge der Rückreflektion, insbesondere aus dem Diffusorkörper zum bzw. in den Lichtleiter, hervorgerufen werden können. Von daher können auch unerwünschte Temperaturerhöhungen des Beleuchtungssystems ausgeschlossen werden, welche sich ungünstig auf eine Behandlung und ggf. auch die Bedienung auswirken können. Eine Gefährdung von Behandelndem und Behandler werden verhindert oder zumindest minimiert.

Eine besonders geeignete Ausführungsform, bei der die feste Umhüllung drei Hüllrohre umfasst, soll nachfolgend dargestellt werden.

Bei dieser Ausführungsform der Erfindung hat sich als besonders vorteilhaft erwiesen, wenn ein erstes Hüllrohr, welches die Matrix zumindest abschnittsweise direkt umgibt, im Wesentlichen klar transparent ausgebildet ist und vorzugsweise einen kleineren Brechungsindex aufweist als der Brechungsindex des Materials der Matrix. Beispielhafte Gläser bzw. deren Zusammensetzung sind in vorstehender Tabelle bzgl. der Mantelgläser aufgeführt.

Dieses erste Hüllrohr kann dabei quasi den optischen Mantel (Cladding) darstellen eines Diffusorkörpers, so dass das eingekoppelte Licht zunächst bis zum distalen Ende des Diffusor-Grundkörpers geführt wird und nur das an den Streuelementen gestreute Licht unter großen Winkeln zur Achse des Diffusor-Grundkörpers seitlich abgestrahlt wird.

Weiterhin vorteilhaft ist ein zweites Hüllrohr vorgesehen, welches das erste Hüllrohr zumindest abschnittsweise umgibt, und wobei vorzugsweise das zweites Hüllrohr transluzent bzw. streuend ausgebildet ist. Dabei kann das zweite Hüllrohr einen größeren Brechungsindex aufweisen als der Brechungsindex des ersten Hüllrohres.

Wenn das zweite Hüllrohr als transluzent bzw. streuend ausgeführt ist und zudem einen Brechungsindex aufweist, welcher größer ist als der des ersten Hüllrohres, kann damit erreicht werden, dass das über das erste Hüllrohr geführte zurück reflektierte Licht definiert ebenfalls nach außen weggestreut werden kann.

Zudem kann infolge einer Mehrfachstreuung an bzw. in diesem zweiten Hüllrohr eine nochmalige Homogenisierung des abgestrahlten Lichts erzielt werden. Das betrifft u.a. auch die Winkelverteilung, so dass eine nahezu Lambert'sche Abstrahlung erzielt werden kann.

Weiterhin vorteilhaft ist ein drittes Hüllrohr vorgesehen, welches das zweite Hüllrohr zumindest abschnittsweise umgibt, wobei vorzugsweise das dritte Hüllrohr im Wesentlichen klar transparent ausgebildet ist.

Mit einem dritten Hüllrohr, welches im Wesentlichen klar transparent ausgebildet ist, kann erreicht werden, dass der Aufbau mechanisch stabilisiert wird und damit auch eine glatte, geschlossene Oberfläche ermöglicht wird.

Je nach Ausgestaltung des zweiten Hüllrohrs kann ein drittes Hüllrohr auch als optional angesehen werden. Dies kann dann zutreffen, wenn, wie weiter oben ausgeführt, dass zweite Hüllrohr auch Weißglas ist oder Weißglas umfasst.

In einer Weiterbildung der Erfindung kann ergänzend vorgesehen sein, dass die feste Umhüllung mehr als drei Hüllrohre umfasst, wobei sich eine Abfolge wie beispielsweise vorstehend erläutert, zum Teil oder vollständig wiederholen kann. Die weiteren Hüllrohre können selbstverständlich aber auch anders ausgebildet sein.

Besonders vorteilhaft ist hierbei, wenn die Matrix mit dem mindestens einem Streuelement und die mehrschichtig bzw. mehrere Hüllrohre umfassende feste Umhüllung als geschlossener Verbund ohne Hohlräume ausgeführt sind.

Dies meint, dass insbesondere eine spaltfreie optische Kopplung zwischen dem ersten und dem zweiten Hüllrohr, vorzugsweise sämtlichen Hüllrohren, erfolgt. Dies bedeutet, dass die jeweiligen Wandungen der Hüllrohre vorzugsweise direkt aneinander anliegen bzw. dass keine Luft, etwa Lufteinschlüsse oder Luftblasen, zwischen den Hüllrohren vorhanden sind. In anderen Worten, die Seitenflächen benachbarter Hüllrohre haben einen möglichst vollflächigen Kontakt zueinander. Hierdurch können sonst unerwünschte optische Effekte hervorgerufen werden, beispielsweise aufgrund der sich dort ergebenden Brechungsindexsprünge.

Wenn, wie dies eine besonders bevorzugte Ausführungsform der Erfindung vorsieht, das Diffusor-Element mit der Matrix, mindestens einem Streuelement und einer festen Umhüllung, welche Hüllrohre aus Glas umfasst, ausgebildet ist, kann ein besonders kompakter, dichter Diffusor-Grundkörper ohne Hohlräume bereitgestellt werden. Hierzu können die Hüllrohre der Umhüllung zu einem kompakten, geschlossenen Körper in einem Ziehprozess verschmolzen werden, wie es weiter unten beschrieben wird.

Auf diese Weise kann sichergestellt werden, dass in der Anwendung keine Flüssigkeit in das erfindungsgemäße Beleuchtungssystem eindringen kann, welche das Abstrahlverhalten negativ beeinflussen könnte.

Zudem ist ein derartiger Diffusor-Grundkörper auch mechanisch sehr stabil. Letzteres kann erreicht werden, wenn die Herstellung in einem Ziehverfahren erfolgt, auf welches weiter unten noch näher eingegangen wird. Bei einem Ziehverfahren können besonders günstig die Viskosität und der thermische Ausdehnungskoeffizient des Materials der Hüllrohre derart ausgewählt und aufeinander abgestimmt werden, dass nach einem Verschmelzen der Komponenten durch den Ziehvorgang bei einer Abkühlung des Diffusor-Grundkörpers, beispielsweise auf Raumtemperatur, eine Druckvorspannung induziert wird. Von Vorteil kann dabei der thermische Ausdehnungskoeffizient des äußersten Hüllrohres derart ausgewählt sein, dass er zumindest etwas über demjenigen des innen angrenzenden Hüllrohres liegt.

Zumindest eines der Hüllrohre, beispielsweise das dritte Hüllrohr, kann in Abhängigkeit von den vorgesehenen Verwendungen gezielt ausgewählt werden, um bestimmte optische Eigenschaften zu bewirken.

So kann in einer Weiterbildung der Erfindung für zumindest ein Hüllrohr, beispielsweise das außenliegende Hüllrohr, zum Beispiel ein Röntgen-opakes Glas ausgewählt werden. Dieses hat den Vorteil, dass der Diffusor-Grundkörper als Ganzes in einem Röntgenbild zumindest teilweise oder abschnittsweise erkennbar ist. Damit ist auf besonders einfache Weise die Position des Diffusors im Körper eines Patienten lokalisierbar.

Im Hinblick auf Anwendungswellenlängen von 0,8 µm bis etwa 2,2 µm, zum Beispiel für die eingangs erwähnten EVLT-Anwendungen, können auch spezielle IR-transparente Gläser, wie sie beispielsweise unten den Bezeichnungen N-PK52a, ein Phosphat-Kron-Glas, oder IRG7, ein Blei-Silikat-Glas, bspw. mit ca. 30 Gew.% PbO, der Anmelderin bekannt sind, zum Einsatz kommen.

Derartige hoch Pb-haltige Gläser bieten zudem den Vorteil, dass diese bei Röntgenbildern entsprechend auffällig sind und als Röntgen-Marker dienen können.

Eine besonders effektive Ausnutzung des in den Diffusor-Grundkörper eingestrahlten Lichtes zur seitlichen Abstrahlung kann mit einer Reflektorfläche erzielt werden, die den Diffusor-Grundkörper am distalen Ende abschließt und/oder zumindest teilweise oder abschnittsweise an dessen Umfangsfläche umfasst, und das Licht direkt und/oder diffus zurück reflektiert.

Dabei haben sich als günstig Reflektorflächen erwiesen, welche als aufgesputterte oder aufgedampfte dielektrische Reflektionsschicht auf dem distalen Ende des Diffusor-Grundkörpers ausgebildet sind, welche aus mehreren Schichten bestehen und hinsichtlich der Reflektivität auf die Wellenlänge des verwendeten Lichtes abgestimmt sind, wobei vorzugsweise ein Maximum der Reflektivität bei dieser Wellenlänge durch die Schichtfolge und die jeweilige Schichtdicke der Einzelschichten einstellbar ist.

Mit derartigen Schichten kann eine Reflektivität von > 95 % bis hin zu > 99,5 % gezielt eingestellt werden.

Alternativ oder zusätzlich kann in einer weiteren Ausführungsform der Erfindung vorgesehen sein, den Reflektor aus einer breitbandig gut reflektierenden Beschichtung, beispielsweise einer Silberschicht, ggf. mit rückseitiger Passivierung bzw. Schutzschicht, auszuführen. Diese sind besonders robust und können störende Reflexe unterdrücken, die zu lokalen Intensitätsüberhöhungen und auch Hotspots führen können. Hiermit kann insbesondere ein sehr breitbandiger Reflektor realisiert werden, der sowohl im sichtbaren Spektralbereich (VIS) als auch im IR/MIR-Bereich ("IR" = Infrarot, "MIR" = mittleres Infrarot), z.B. zwischen 1 µm und 2,5 µm Wellenlänge, sehr gute Reflektionseigenschaften aufweist. Eine rückseitige Passivierung verhindert eine Oxidation z.B. der Silberschicht.

In einer weiteren Ausführungsvariante kann vorgesehen sein, dass ein erstes Maximum der Reflektivität für eine erste Wellenlänge, beispielsweise die Wellenlänge des verwendeten Lichtes bzw. die Anwendungswellenlänge, und zumindest ein weiteres, zweites Maximum der Reflektivität auch für mindestens eine weitere Wellenlänge besteht. Die zumindest eine weitere Wellenlänge kann sich dabei vorteilhaft von der Anwendungswellenlänge unterscheiden. In höchst vorteilhafter Weise lassen sich auf diese Weise weitere Funktionen integrieren.

Dabei können die zumindest zwei Maxima insbesondere eine Reflektivität von > 95 %, bevorzugt von > 99 % aufweisen. In anderen Worten, ein erstes Maximum der Reflektivität kann bei einer bestimmten Wellenlänge des Lichtes der Lichtquelle und zumindest ein weiteres, zweites Maximum der Reflektivität bei einer weiteren Wellenlänge bestehen, wobei sich diese Wellenlänge von der ersten Wellenlänge des Lichtes der Laser-Lichtquelle unterscheiden kann, und wobei die Reflektivität des ersten Maximums und die des zumindest einen weiteren Maximums vorzugsweise > 95 %, bevorzugt > 99 % beträgt. Zur Erzeugung von Licht der weiteren Wellenlänge kann beispielsweise eine weitere Lichtquelle, etwa eine weitere LED-Lichtquelle, vorgesehen sein, welche beispielsweise Licht mit geringerer Leistung abgibt.

Der Begriff Anwendungswellenlänge meint hierbei die Wellenlänge elektromagnetischer Strahlung, welche für eine bestimmte Behandlung vorgesehen oder ausgewählt ist, z.B. 690 nm.

Damit können störende Abstrahlungen am distalen Ende des Diffusor-Grundkörpers für die Anwendungswellenlänge, z.B. 690 nm, hier gemeint die Behandlungswellenlänge, sowie für eine andere Wellenlänge, z.B. die eines Pilotlichtes mit beispielsweisem grünen Licht, z.B. 500 nm bis 550 nm, welches oft zum Einrichten und zur Funktionskontrolle des Bauteils verwendet wird, vermieden werden.

In einer weiteren Ausbildungsform der Erfindung ist vorgesehen, dass die Reflektorfläche zumindest abschnittsweise unter einem Winkel von kleiner 90°, bevorzugt zwischen 85,0° bis 89,9° zur Längsachse des Diffusor-Grundkörpers ausgebildet ist und im Betrieb rückreflektiertes Licht mit einer größeren numerischen Apertur NA reflektierbar ist als das Licht, welches auf die Reflektorfläche trifft.

Damit kann eine bessere Nutzung der im Diffusor-Grundkörper eingelagerten Streuelemente zur seitlichen Lichtauskopplung erreicht werden, was mit einer Effizienzsteigerung, aber auch mit einer Erhöhung der Homogenität über die Diffusorlänge einhergeht.

In nochmals weiteren Ausführungsformen kann eine konkav oder konvex ausgebildete Reflektorfläche vorgesehen sein. Besonders bevorzugt hinsichtlich des Herstellprozesses können auch einfach schräg zur Längsachse des Diffusor-Grundkörpers ausgebildete plane Reflektorflächen oder eine facettierte Reflektorfläche sein, welche abschnittsweise plane Flächen aufweist, welche unterschiedliche Winkel zur Längsachse des Diffusor-Grundkörpers aufweisen

Trotz der zuvor genannten Maßnahmen kann unter bestimmten Umständen immer noch ein Teil des zurück reflektierten Lichtes, insbesondere im Bereich der als mechanischen Verstärkung vorgesehenen transparenten Hülse am Übergang zwischen Diffusor-Grundkörper und Lichtleiter, über diese Hülse in Richtung des zuführenden Lichtleiters und/oder in diesen hinein geführt werden, was zum einen ein unerwünschtes Aufleuchten des Lichtleiters hervorrufen kann, oder aber, etwa bei größeren Lichtleistung, zu einer unerwünschten Erwärmung führen.

Die transparente Hülse kann aus Kunststoff, Glas, Metall oder keramischen Werkstoff hergestellt sein oder Kunststoff, Glas, Metall oder keramischen Werkstoff umfassen. Der Kunststoff kann beispielsweise ausgewählt sein aus der Gruppe der thermoplastischen Kunststoffe wie Polycarbonat (PC). Glas bietet aufgrund seines E-Moduls Vorteile in Bezug auf die mechanische Stabilität.

Eine Erwärmung kann insbesondere auch lokal als sogenannter Hotspot infolge von lokalen Absorptionen erzeugt werden. Insbesondere wenn das über die transparente Hülse geführte Licht auf dem äußeren Mantel (Buffer) des Lichtleiters trifft, führt dies zu einem intensiv ausgeprägten Lichtreflex und zur lokalen Erwärmung.

Es ist daher in einer besonders bevorzugten Ausführungsform der Erfindung vorgesehen, dass im Bereich zwischen der transparenten Hülse und dem äußeren Mantel des Lichtleiters eine weitere transluzente bzw. teilabsorbierende Hülle vorgesehen ist.

Diese transluzente bzw. teilabsorbierende Hülle kann von Vorteil aus einem Polymer, in welches Streupartikel eingelagert sind, ausgebildet sein.

Damit kann zumindest eine teilweise Reduktion des auf den äußeren Mantel auftreffenden Lichts erreicht werden. Durch Mehrfachreflektion in dieser Hülle kann es zudem zu einem seitlichen Abstrahlen kommen, was aber kaum noch mit dem Auge wahrnehmbar ist, da dies über die komplette Länge dieser Hülle gleichmäßig und mit deutlich geringerer Leistungsdichte geschieht, so dass dieses seitliche Abstrahlen deutlich abgeschwächt erfolgt. Hotspots werden damit vermieden.

Hinsichtlich der technischen Umsetzung hat es sich als vorteilhaft erwiesen, wenn die transluzente bzw. teilabsorbierende Hülle ein Schlauchabschnitt, ein Schrumpfschlauchabschnitt und/oder ein Re-Coating-Polymer ist, in das Streupartikel zuvor einbringbar sind, sofern dies gewünscht ist.

Im Rahmen der Erfindung liegt auch ein Verfahren zur Herstellung eines erfindungsgemäßen Diffusor-Grundkörpers bzw. eines erfindungsgemäßen Beleuchtungssystems, vorzugsweise mit einer dem Anwendungszweck angepassten Beleuchtungsprofil, insbesondere der Homogenität der Intensität der im Betriebszustand seitlichen Abstrahlung.

Es wird ein Verfahren angegeben zum Herstellen eines erfindungsgemäßen Diffusor-Grundkörpers, insbesondere für ein erfindungsgemäßes Beleuchtungssystem, umfassend mindestens ein Streuelement, wobei vorzugsweise das zumindest eine Streuelement entlang der Längsachse eines Diffusor-Grundkörpers im Wesentlichen zu diesem parallel ausgerichtet ist oder in einem Winkel zur Längsachse des Diffusor-Grundkörpers angeordnet ist, beinhaltend die Verfahrensschritte:
- Bereitstellen einer Mehrzahl von Lichtleitstäben aus einem Glas mit dem Brechungsindex n₁ und/oder n₁',
- Anordnen der Mehrzahl der Lichtleitstäbe mit dem Brechungsindex n₁ und/oder n₁' und zumindest eines Streustabs aus einem Glas oder einer Glaskeramik, die Streuzentren umfasst, so dass die Längsachsen der Lichtleitstäbe und des zumindest einen Streustabs im Wesentlichen, parallel zueinander verlaufen und eine Vorform erhalten wird,
- Erwärmen der Vorform,
- Ausziehen der Vorform in einer Ziehanlage und ggf. Ablängen, um einen Diffusor-Grundkörper zu erhalten.

Bei dem auf diese Weise erhaltenen Diffusor-Grundkörper verbinden sich die Außenumfangsflächen der Lichtleitstäbe, unlösbar, formschlüssig miteinander und mit dem zumindest einem Streustab. Insbesondere verschmelzen sie miteinander und bilden somit die Matrix des Diffusor-Grundkörpers mit zumindest einem eingelagerten und/oder an diesen angrenzendem Streuelement, gebildet aus dem zumindest einen ausgezogenen Streustab.

Es werden somit eine Mehrzahl von Lichtleitstäben aus einem Glas mit dem Brechungsindex n₁ bzw. n₁' bereitgestellt.

Abhängig von dem zu erreichenden Beleuchtungsprofil werden zumindest einer oder mehrere Streustäbe aus einem Glas oder Glaskeramik beinhaltend die beschriebenen Streuzentren in einer erforderlichen Anzahl bereitgestellt und neben beziehungsweise zwischen den Lichtleitstäben angeordnet, so dass eine Anordnung von Lichtleitstäben und Streustäben entsteht, in der die Längsachsen von Lichtleitstäben und Streustäben vorteilhaft im Wesentlichen parallel zueinander angeordnet werden. Die Verteilung der Streustäbe in der Anordnung kann nach einem von dem gewünschten Beleuchtungsprofil abhängigen Muster erfolgen. Diese Anordnung wird durch geeignete Mittel fixiert und bildet so eine Vorform.

In einem folgenden Verfahrensschritt wird die Vorform erwärmt und zu einem seitlich abstrahlenden Glaselement so ausgezogen, so dass sich die Lichtleitstäbe und der zumindest eine Streustab an ihren Außenumfangsflächen unlösbar miteinander verbinden. Die Temperaturführung beim Ausziehen bewirkt auch, dass zwischen den Lichtleitelementen eine Phasengrenze vorhanden bleibt. Dies kann insbesondere dadurch erreicht werden, dass die Ziehtemperatur unterhalb der Schmelztemperatur des Glases der Lichtleitstäbe gehalten wird und diese insbesondere bei der Sintertemperatur zusammengesintert werden. Eine vollständige Verschmelzung der Lichtleitstäbe wird erfindungsgemäß vermieden. Ebenso durch die Temperaturführung wird der bevorzugte Formschluss der Lichtleitstäbe und bedarfsweise auch der Streuelemente erreicht.

Das so erhaltene Glaselement kann direkt den Diffusor-Grundkörper bilden. Insbesondere kann aber auch der Diffusor-Grundkörper und oder Abschnitte dessen durch Konfektionieren, beispielsweise Ablängen, des hergestellten Glaselementes erhalten werden. Die Matrix des Diffusor-Grundkörpers wird dabei aus den ausgezogenen, formschlüssig verbundenen Lichtleitstäben gebildet, in welche das zumindest eine Streuelement mit den Streuzentren, das aus den ausgezogenen Streustäben gebildet wird, ebenfalls formschlüssig, im Wesentlichen entsprechend dessen Anordnung in der Vorform, eingelagert ist.

In einer vorteilhaften Ausführungsform sind die Lichtleitstäbe wie beschrieben nicht vollständig miteinander verschmolzen, und auch der Streustab ist nicht vollständig mit zumindest einem der Lichtleitstäbe verschmolzen. Eine Phasengrenze kann dann auch zwischen dem Streustab und den Lichtleitstäben vorhanden sein und verbleibt somit auch innerhalb der gebildeten Matrix und den Streuelementen des Diffusor-Grundkörpers. Diese Ausführungsform lässt sich dadurch erreichen, dass die Erweichungstemperatur des Glases der Lichtleitstäbe gleich hoch oder niedriger als die Erweichungstemperatur der Streustäbe ist.

Eine ebenso vorteilhafte Ausführungsform sieht vor, dass die Lichtleitstäbe nicht vollständig miteinander verschmolzen und eine Phasengrenze zwischen diesen vorhanden ist, aber das zumindest eine Streuelement auf zumindest einen Lichtleitstab aufgeschmolzen ist. Dies kann erreicht werden, indem die Erweichungstemperatur des Glases der Streustäbe kleiner als die des Glases der Lichtleitstäbe gewählt wird. Vorteilhaft hat sich eine bis zu 50 K niedrigere Erweichungstemperatur des Glases der Streustäbe erwiesen, insbesondere eine bis zu 30 K niedrigere Erweichungstemperatur.

Beim Ausziehen werden aus den Lichtleitstäben die Matrix und aus den Streustäben die Streuelemente des Glaselements. Die Lichtleitstäbe bestehen daher aus einem Glas mit einem Brechungsindex n₁ und sind im Einzelnen nicht von einem Mantelglas mit dem Brechungsindex n₂ umhüllt.

Die Mittel zum Fixieren der Anordnung der Vorform aus Lichtleitstäben und Streustäben können beispielsweise Klammern sein, die nachher wieder entfernt werden.

Die auf diese Weise erhaltene Matrix mit dem zumindest einem Streuelement wird in einem weiteren Ziehprozess mit der festen Umhüllung versehen. Im Sinne der Erfindung wird vorzugsweise eine feste Umhüllung verwendet, welche mit einem mehrteiligen oder mehrschichtigen Aufbau ausgebildet sein kann. In einer bevorzugten Ausführungsform umfasst diese feste Umhüllung zumindest zwei, besonders bevorzugt zumindest drei Hüllrohre.

Zum Herstellen einer festen Umhüllung mit zwei Hüllrohren kann die Matrix mit dem zumindest einem Streuelement im Innern des ersten Hüllrohres angeordnet werden. Das erste Hüllrohr kann sodann in dem zweiten Hüllrohr platziert werden.

Im Einzelnen kann das Verfahren die folgenden Schritte umfassen:
- Bereitstellen einer Matrix mit dem zumindest einem Streuelement,
- Schaffen einer Anordnung, bei welcher die Matrix mit dem zumindest einem Streuelement im Innern des ersten Hüllrohres angeordnet wird,
- Platzieren des ersten Hüllrohres in einem zweiten Hüllrohr,
- Ausziehen der Anordnung in einer Ziehanlage und ggf. Ablängen, um einen Diffusor-Grundkörper mit fester Umhüllung zu erhalten.

Zusätzlich kann beim Ausziehen der Anordnung in einer Ziehanlage die Vorform um die Längsachse der Vorform verdreht werden, um damit beispielsweise eine spiralförmige, bevorzugt Helix-förmige Anordnung der Streuzentren um die Längsachse zu erzeugen.

Zum Herstellen einer festen Umhüllung mit drei Hüllrohren kann die Matrix mit dem zumindest einem Streuelement im Innern des ersten Hüllrohres angeordnet werden. Das erste Hüllrohr kann sodann in dem zweiten Hüllrohr plaziert werden, und dieses wiederum in dem dritten Hüllrohr.

Im Einzelnen kann das Verfahren daher noch den folgenden Schritt umfassen:
- Plazieren des zweiten Hüllrohres in einem dritten Hüllrohr, bevor die Anordnung in einer Ziehanlage ausgezogen wird.

Die Reihung der Zusammenstellung kann selbstverständlich auch anders gestaltet sein, sofern Lichtleitstäbe und Streustäbe, erstes, zweites oder auch drittes Hüllrohr entsprechend vormontiert sind. Diese Anordnung kann sodann einem Ziehprozess in einer Ziehanlage zugeführt werden, um diese Komponenten zu einem kompakten, geschlossenen Körper miteinander zu verschmelzen, welcher die Basis für das gewünschte Diffusor-Element darstellen kann.

Zumindest eines der Hüllrohre kann dabei einseitig verschlossen sein, was den Zusammenbau und das Schaffen der Anordnung erleichtert.

Überraschenderweise lassen sich auch derartige vormontierte Anordnungen mit drei Hüllrohren oder sogar noch mehr Hüllrohren sowie einer Matrix mit zumindest einem Streuelement im Inneren als Ganzes in einem Ziehprozess in einem einzigen Schritt zu dem gewünschten Diffusor-Element formen. Beim Erwärmen und Ausziehen erweichen die Hüllrohre und legen sich auf die jeweils weiter innen angeordnete Komponente, so dass quasi ein dreifacher Mantel um die Matrix mit Streuelement gebildet wird.

Das durch das Erwärmen und Ausziehen erhaltene Produkt kann anschließend zerteilt oder abgelängt und/oder entsprechend weiterverarbeitet werden.

Durch Variation der Parameter Geschwindigkeit, Temperatur und/oder Kraft im Ziehprozess können, gegebenenfalls nach einer Konfektionierung, zumindest teilweise oder abschnittsweise kegelförmige, sich verjüngende Diffusor-Grundkörper erhalten werden. Zumindest im Bereich einer Verjüngung verlaufen dann die Streuelemente nicht mehr parallel zur Längsachse des Diffusor-Grundkörpers, sondern in einem Winkel zu dieser.

Eine bevorzugte Verwendung des Beleuchtungssystems, wie es zuvor in seinen verschiedenen Ausführungsvarianten beschrieben wurde, sieht den Einsatz für eine photodynamische Therapie (PDT) oder Photo-Immuno-Therapie (PIT) beispielsweise zur Tumortherapie, für eine endovenöse Lasertherapie (EVLT) beispielsweise zur Behandlung von Krampfadern, für eine Laser-induzierte interstitielle Thermotherapie (LITT) beispielsweise zur Behandlung von Epilepsie oder Gehirntumoren oder für Anwendungen im Bereich der Dentalmedizin, Augenheilkunde sowie Dermatologie vor, wie diese eingangs beschrieben wurden. Im Bereich Dentalmedizin sind hier insbesondere Applikationen zur Wund- oder Parodontose-Behandlung zu nennen. Darüber hinaus gibt es Anwendungen in der Hirnforschung, bei denen mittels Licht einzelnen Gehirnbereich stimuliert und damit Krankheitssymptome behandelt werden können.

Eine weitere Verwendung des Beleuchtungssystems, wie es zuvor in seinen verschiedenen Ausführungsvarianten beschrieben wurde, sieht den Einsatz für eine photodynamische Therapie (PDT) zur Tumortherapie vor, wobei zumindest ein Lichtleiter mit dem Diffusor-Element aus anderen Diffusor-Elementen abgestrahltes Licht aufnimmt und über den Lichtleiter einem Detektor zur spektroskopischen Analyse weiterleitet. Dabei werden dem Patienten neben den verschiedenen Lichtaussendenden Diffusor-Lichtleitern auch lichtempfangende Diffusor-Lichtleiter appliziert, wobei anhand der spektralen Unterschiede zwischen eingekoppeltem und empfangenen Licht auf ein Ansprechen der PDT-Behandlung geschlossen werden kann (siehe dazu Finlay et al., Proc. SPIE Int. Soc. Opt. Eng. 2014, June 14; 5315: Page 132-142). Zudem können damit auch Dosimetrische Aufgaben realisiert werden.

Darüber hinaus sind auch Anwendungen im industriellen Bereich vorteilhaft, etwa zur Inspektion von schwer zugänglichen Stellen beispielsweise an oder in einer Maschine, bei denen es insbesondere auf eine homogene Ausleuchtung ankommt, oder auch spektroskopische Anwendungen oder in der Biochemie, bei der durch Licht biochemische In-Vitro-Reaktionen stimuliert werden.

Die Erfindung wird im Folgenden anhand eines in den Figuren dargestellten Ausführungsbeispiels näher erläutert. Es zeigen:
- Fig. 1: schematisch ein Beleuchtungssystem mit einem Lichtleiter und einem Diffusor-Element in einer PDT-Anwendung,
- Fig. 2: in einer schematischen Längsschnitt-Darstellung das Diffusor-Element,
- Fig. 3: in einer schematischen Querschnitt-Darstellung ein bevorzugtes Ausführungsbeispiel für die Anordnung von Streuelementen in einem Diffusor-Grundkörper,
- Fig. 4: in einem Verlaufsdiagram einen Intensitätsverlauf,
- Fig. 5: in einer schematischen Darstellung einen Aufbau zur Vermeidung von rückstreuenden Licht in den äußeren Mantel des Lichtleiters,
- Fig. 6a, 6b, 6c, 6d: verschiedene Ausführungsbeispiele für die Anordnung von Streuelementen in einem Diffusor-Grundkörper,
- Fig. 7a und 7b: verschiedene Ausführungsbeispiele für Streuelemente in einer Matrix des Diffusor-Grundkörpers,
- Fig. 8a, 8b und 8c: schematisch verschiedene Ausgestaltungsbeispiele einer Reflektorfläche des Diffusor-Grundkörpers, und
- Fig. 9: eine schematische Darstellung eines Diffusor-Grundkörpers mit einer Helix-förmigen Anordnung der Streuzentren um die Längsachse des Diffusor-Grundkörpers

### Detaillierte Beschreibung bevorzugter Ausführungsformen:

Bei der nachfolgenden Beschreibung der detaillierten Ausführungsformen bezeichnen gleiche Bezugszeichen in den beiliegenden Figuren jeweils gleiche oder gleich wirkende Bestandteile.

Zum besseren Verständnis werden die nachfolgenden Definitionen vorgenommen.

Im Sinne der vorliegenden Offenbarung umfasst der Begriff des Beleuchtungssystems Beleuchtungsvorrichtungen und insbesondere Beleuchtungsvorrichtungen, welche zur Anwendung für medizintechnische Zwecke geeignet und insbesondere, soweit diese mit lebendem Gewebe in Kontakt treten sollen, zumindest abschnittsweise desinfizierbar und/oder sterilisierbar sind.

Die Aussage "für ein medizintechnisches Therapie- und/oder Diagnosesystem" umfasst auch die Verwendung oder Anwendung des hier offenbarten Beleuchtungssystems selbst als medizinisches Therapie- und/oder Diagnosesystem.

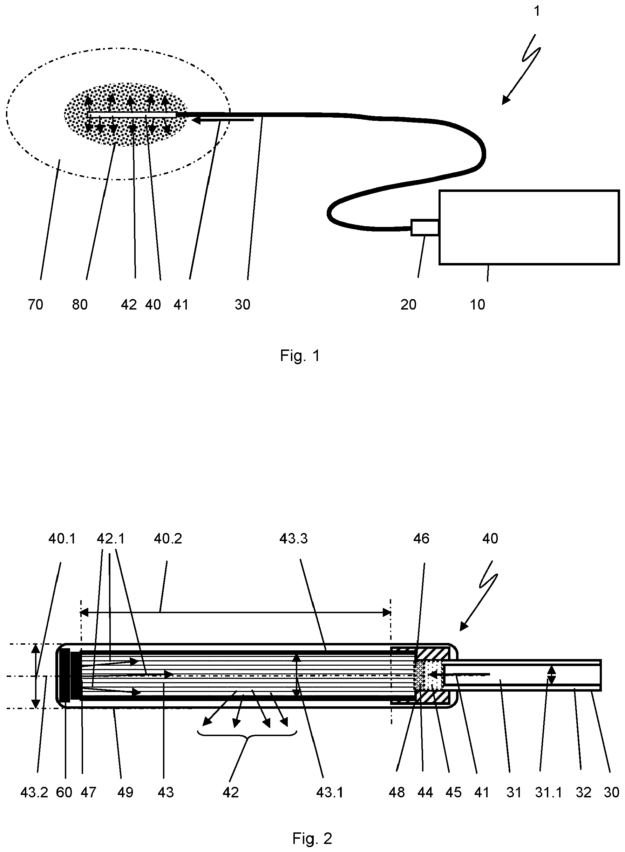

Fig. 1 zeigt schematisch den Aufbau eines Beleuchtungssystems 1 gemäß der einer bevorzugten Ausführungsform der Erfindung. Beispielhaft ist hier eine medizintechnische PDT-Anwendung dargestellt.

Im gezeigten Beispiel umfasst das Beleuchtungssystem 1 eine Lichtquelle 10, insbesondere eine Laser-Lichtquelle, welche im Betriebszustand Licht in einem bestimmten Spektralbereich aussendet. Für PDT-Anwendungen, wie sie eingangs beschrieben sind, werden Laser verwendet, die auf den zuvor verabreichten biochemisch modifizierten Farbstoff (Photosensitizer) abgestimmte Wellenlänge üblicherweise im sichtbaren Bereich, beispielsweise im grünen Spektralbereich bei 532 nm oder im roten Spektralbereich bei zum Beispiel 690 nm, aussenden. Hier sei angemerkt, dass grundsätzlich auch LED- oder LD-basierte Lichtquellen zum Einsatz kommen können. Hinsichtlich der erzielbaren Leistungsdichten haben sich hier allerdings Laser-basierte Systeme durchgesetzt.

Ein Lichtleiter 30 ist mit einem Stecker 20 an seinem proximalen Ende an die Lichtquelle 10 angeschlossen. Als proximales Ende wird hierbei das Ende des Lichtleiters 30 bezeichnet, in welches Licht eingekoppelt wird. Am distalen Ende weist der Lichtleiter 30 ein Diffusor-Element 40 auf, welches über hier nicht dargestellte Kanülen in ein Tumor-Gewebe 80 eingebracht werden kann, welches sich innerhalb eines gesunden Gewebes 70 gebildet hat. Der Wirkbereich des Diffusor-Elementes entspricht im Idealfall dem Bereich des Tumor-Gewebes 80.

Als distales Ende wird hierbei das andere Ende des Lichtleiters 30 bezeichnet, welches in der Regel zu dem proximalen Ende des Lichtleiters 30 entfernt angeordnet ist und aus welchem insbesondere Licht austritt.

Die Laserstrahlung gelangt dabei über eine Lichteinkopplung 41 am Diffusor-Element 40 in das Diffusor-Element 40 und wird über die Länge des Diffusors seitlich abgestrahlt (Lichtauskopplung 42). Dabei kommt es auf eine möglichst homogene Abstrahlung über die Länge des Diffusor-Elementes 40 an. Insbesondere sind Intensitätsspitzen zu vermeiden. Durch eine photoinduzierte biochemische Reaktion, wie sie eingangs beschrieben ist, kommt es nach der Behandlung idealerweise zu einem Absterben des Tumor-Gewebes 80.

In der Regel werden als Lichtleiter 30 Quarz-Fasern verwendet, wobei die Stecker 20 in der Regel als koaxialer Steckverbinder, sogenannte SMA-Stecker, ausgebildet sind, bei denen die Fasern in den Stecker 20 geklebt sind. Vorteilhaft hinsichtlich der thermischen Belastbarkeit können auch Stecker 20 mit Neusilber-Hülsen sein, bei denen der Lichtleiter 30 in die Neusilber-Hülse formschlüssig durch plastische Verformung eingebracht, gecrimpt, ist. Darüber hinaus können bei größeren Laser-Leistungen auch Stecker 20 zum Einsatz kommen, bei denen das Faserende des Lichtleiters 30 durch ein Kegelprisma geschützt ist, was vorteilhaft bei Fehljustagen sein kann. Der Lichtleiter kann, wie oben beschrieben eine Einzelfaser, beispielsweise eine Mono- oder Multimodelichtleitfaser, umfassend einen Kern mit einem Kerndurchmesser und einen Mantel, oder ein Faserbündel mit einem Faserbündeldurchmesser umfassen.

Fig. 2 zeigt schematisch den Aufbau eines Diffusor-Elementes 40 gemäß einer bevorzugten Ausführungsform der Erfindung.

Das Diffusor-Element 40 besteht aus einem Diffusor-Grundkörper 43, welcher über eine Verbindungszone 44 an den Lichtleiter 30 befestigt ist. Der Lichtleiter 30 besteht bei den zuvor beschriebenen Anwendungen meist aus Quarzglas mit einem Kern 31 mit einem Brechungsindex n₁ und einem Kerndurchmesser 31.1 von üblicherweise zwischen 200 und 600 µm sowie einem Mantel 32 mit einem Brechungsindex n₂, wobei gilt n₁ > n₂. Die damit üblicherweise erzielbare numerische Apertur NA beträgt etwa 0,22. Die Lichteinkopplung 41 erfolgt über Einkoppelfläche 46 des Diffusor-Grundkörpers 43. Üblicherweise weist der Lichtleiter 30 noch einen äußeren Mantel 33 auf, üblicherweise aus einem Polymer bestehend, z.B. PMMA, PA (z.B. NYLON^{®}) oder einem fluorierten Polymer (z.B. TEFZEL^{®)}, welcher auch als Buffer bezeichnet wird. Dieser ist in Fig. 2 nicht dargestellt.

Der Diffusor-Grundkörper 43 mit seinem Durchmesser 43.1 umfasst bei einer bevorzugten Ausführungsform eine feste Umhüllung 43.3 und eine Matrix 43.4 aus Matrix-Elementen 43.5 mit eingelagerten Streuelementen 43.6 oder besteht aus einer festen Umhüllung 43.3 und einer Matrix 43.4 aus Matrix-Elementen 43.5 mit eingelagerten Streuelementen 43.6.

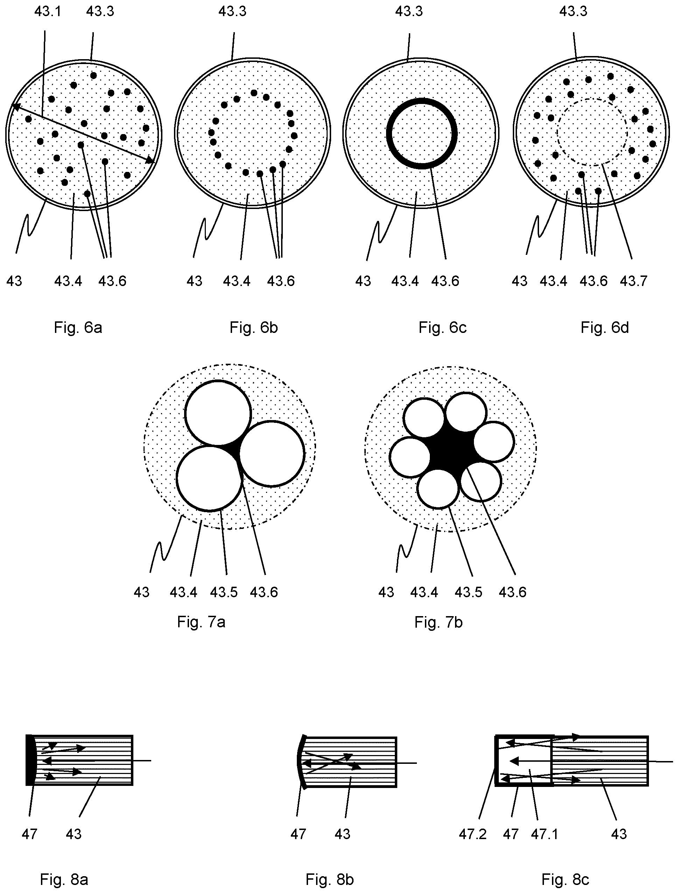

Rein beispielhaft sind in den Fig. 6a, 6b, 6c und 6d verschiedene Ausführungsbeispiele für günstige Anordnungen von Streuelementen 43.6 in einem Diffusor-Grundkörper 43 dargestellt. Ferner sind in den Fig. 7a und 7b verschiedene Ausführungsbeispiele für Streuelemente in einer Matrix 43.4 des Diffusor-Grundkörpers 43 dargestellt. Auf diese Ausführungsbeispiele wird weiter unten vertiefend eingegangen.

Erfindungsgemäß kann die feste Umhüllung 43.3 aus mehreren, vorzugsweise zumindest zwei, koaxial zueinander angeordneten Hüllrohren bzw. Schichten 43.3.1, 43.3.2, 43.3.3 bestehen oder diese umfassen, welche unterschiedliche optische Eigenschaften in Bezug auf zumindest die Transparenz, den Brechungsindex und/oder das Material des Hüllrohrs aufweisen können.

Um im Betriebszustand die Homogenitätsanforderungen hinsichtlich der Intensität der seitlichen Abstrahlung erfüllen zu können, umfasst der Diffusor-Grundkörper 43 abhängig von der Betriebswellenlänge und der Diffusorlänge zwischen 10 und 100 Streuelemente 43.6. Als Faustregel gilt hier: Je größer die Anwendungswellenlänge bzw. je kürzer die Diffusorlänge, desto mehr Streuelemente 43.6 sind vorzusehen.

Das Verhältnis der Querschnittsflächen von eingelagerten Streuelementen 43.6 und Diffusor-Grundkörper 43 ergibt sich zu ≤ 0,015, bevorzugt ≤ 0,005, besonders bevorzugt ≤ 0,002. Die Streuelemente 43.6 sind dabei über die komplette Länge des Diffusor-Grundkörpers 43 im Wesentlichen parallel zur Längsachse 43.2 ausgerichtet.

In vorteilhafter Ausgestaltung ist der Durchmesser des Diffusor-Grundkörper 43 grösser als der Kerndurchmesser 31.1 oder Faserbündeldurchmesser 31.1 des Lichtleiters 30 ausgelegt, so dass einerseits kein unkontrolliertes Streulicht beispielsweise in die feste Umhüllung 43.3 eingekoppelt wird. Andererseits können damit Montage und die Justage von Lichtleiter 30 und Diffusor-Grundkörper 43 erleichtert und/oder Montagetoleranzen ausgeglichen werden. Das Verhältnis des Kerndurchmessers 31.1 oder Faserbündeldurchmessers 31.1 des Lichtleiters 30 und des Durchmessers des Diffusor-Grundkörpers 43.1 mit den eingelagerten Streuelementen 43.6 beträgt damit vorteilhaft ≤ 1,0, bevorzugt zwischen 1,0 und 0,8. Je nach gewünschter Abstrahlcharakteristik kann auch Verhältnis von ≤ 0,8 vorgesehen sein.

In der Verbindungszone 44 zwischen dem proximalen Ende des Diffusor-Grundkörpers 43 und dem distalen Ende des Lichtleiter 30 kann ein optisches Element angeordnet sein, das beispielsweise als Strahlformungselement, Lichtleitelement oder faseroptischer Taper, gegebenenfalls konisch ausgebildet sein kann. So werden auch geometrische Anpassung beispielsweise von Durchmesserunterschieden ermöglicht. Hierbei bezeichnet das proximale Ende des Diffusor-Grundkörpers 43 das Ende des Diffusor-Grundkörpers 43, in welches Licht eingekoppelt wird.

Zur Vermeidung von Streulicht aus der Verbindungszone 44 aber auch als mechanische Stabilisierung der Verbindungszone 44 ist gemäß einer bevorzugten Ausführungsform der Erfindung eine Hülse 48 aus Kunststoff, Glas, Metall oder keramischen Werkstoff vorgesehen, durch welche Licht aus dem Lichtleiter 30 in der Richtung der Längsachse des Lichtleiters 30 sowie unter bestimmten seitlichen Winkeln hindurchtreten kann, jedoch Licht abgeschattet wird, welches stirnseitig in das proximale Ende des Streukörpers eintreten kann. Als besonders wirksam hinsichtlich einer Vermeidung von Licht-Absorptionen hat sich eine Hülse 48 aus einem borosilikatartigem Glas erwiesen. Ein derartiges Glas ist z.B. unter der Bezeichnung SCHOTT FIOLAX^{®} 8412 des Herstellers Schott AG bekannt.

Am distalen, dem proximalen Ende gegenüberliegenden Ende des Diffusor-Grundkörpers 43 ist zur Optimierung der Abstrahlcharakteristik eine Reflektorfläche 47 vorgesehen, welche gerichtet reflektierend als Spiegelelement in Form eines Metallblättchens oder als dünne Spiegelfolie beispielsweise einer Trägerfolie mit aufgedampfter Spiegelschicht oder einer Beschichtung mit einer Reflektivität > 95 % ausgebildet sein kann. Als vorteilhaft hat sich auch eine diffus reflektierende Schicht beispielsweise mittels Aufbringung zum Beispiel Bedruckung mit bevorzugt weißer Farbe, herausgestellt.

In einer weiteren Ausgestaltungsvariante kann vorgesehen sein, dass die Reflektorfläche 47 als kurze polierte Drahtabschnitte aus Aluminium oder Gold hergestellt sind, die direkt in Kontakt mit dem Diffusor-Grundkörper 43 gebracht sind. Damit ergeben sich zudem kleine Wärmesenken, die Hotspots vermeiden helfen.

Weiterhin haben sich als besonders vorteilhaft aufgesputterte oder aufgedampfte dielektrische Reflektionsschichten auf dem distalen Ende des Diffusor-Grundkörpers 43 herausgestellt, welche aus mehreren Schichten bestehen und hinsichtlich der Reflektivität auf die Wellenlänge des verwendeten Lichtes abgestimmt sind. Geometrische Ausführungsformen von Reflektorflächen 47 werden weiter unten beschrieben. Auf diese Weise lässt sich beispielsweise eine Reflektivität von > 95 %, bevorzugt > 99 % erreichen.

Der Begriff "hinsichtlich der Reflektivität auf die Wellenlänge des verwendeten Lichtes abgestimmt" gibt im Rahmen der vorliegenden Offenbarung an, dass beim Abstimmen eine möglichst hohe Reflektivität bei dieser Wellenlänge erreicht wird oder sogar das Maximum der Reflektivität bei der Wellenlänge liegt, auf welche jeweils abgestimmt wird. Beispiele einer solchen Reflektorschicht ist ein Mehrfach-Schichtsystem aus alternierend aufgebrachten TiO₂- und SiO₂-Schichten, welches z.B. eine Reflektivität von > 99 %, vorzugsweise > 99,5 %, im Anwendungswellenlängenbereich, z.B. für rotes Licht mit (690 ± 10) nm, aufweist.

Derartige Schichtsysteme können auf die jeweilige Anwendungswellenlänge entsprechend angepasst, dies bedeutet wie vorstehend angegeben, abgestimmt werden. Hiermit kann eine ideale Rückreflektion einerseits und eine Vermeidung von Hotspots andererseits erreicht werden. Alternativen dazu oder ergänzend können auch Silberschichten mit rückseitiger Passivierung als Reflektorfläche 47 vorgesehen sein. Ebenso denkbar ist ein Schutz der Reflektorschicht am distalen Ende mittels beispielsweise eines Klebertropfens, der dann eine abgerundete Kappe im ausgehärtetem Zustand ausbildet.

Da in einer PDT- oder PIT-Anwendung oft ein sogenanntes Pilotlicht zum Einrichten, d.h. zum Positionieren des Diffusors beispielsweise in vivo, verwendet wird, welches sich hinsichtlich der Leistung und vor allem der Wellenlänge deutlich von der Anwendungswellenlänge (z.B. 690 nm) unterscheidet, kann einerseits ein zusätzliches Blockierelement 60 beispielsweise in Form eines Keramik- oder Metallzylinders, einer Keramik- oder Metallkugel in distaler Richtung gesehen hinter der Reflektorfläche 47 vorgesehen sein und/oder das Schichtsystem der Reflektorfläche 47 ist derart ausgelegt, dass zusätzlich auch eine hohe Reflektivität von > 80 %, bevorzugt > 95 %, besonders bevorzugt > 99% für die Wellenlänge des Pilotlichtes, beispielsweise im grünen Spektralbereich zwischen 500 nm und 580 nm, erzielt werden kann.

Metallische Blockierelemente 60 haben zudem den Vorteil, dass sie als sogenannte Röntgen-Marker oder Radiomarker im Röntgenbild erkannt werden können. Dies trifft auch für die Hülse 48 oder einer zusätzlichen Hülse im Bereich der Verbindungszone 44 zu, wenn diese als dünnwandige Metallhülse ausgelegt ist. Typischerweise sind derartige Metallhülsen aus einem Material oder Materialien, einer Kombination oder Legierung daraus, mit hoher Ordnungszahl gefertigt. Beispiele dafür sind Tantal, Platin, Iridium oder Platin-Iridium-Legierungen.

Zum weiteren mechanischen Schutz und/oder zur Homogenisierung der Abstrahlcharakteristik kann eine Hülle 49 aus transparenten und/oder transluzentem, eingefärbten oder farblosen Material (Silikon, Glas oder Quarzglas) vorgesehen sein, die den Diffusor-Grundkörper zumindest teilweise oder abschnittsweise umschließt. Insbesondere kann mit einem transluzenten und/oder Streuzentren enthaltenden Material eine zusätzliche Homogenisierung erzielt werden. Geeignet sind beispielsweise entsprechende Körper oder Schläuche aus Silikon, Teflon oder auch aus einem Polyether-Block-Amid-Block-Copolymer, welches beispielsweise als PEBAX^{®} im Handel bekannt ist. Als Hülle 49 haben sich, zumindest abschnittsweise aufgebrachte, dünnwandige Schrumpfschläuchen, z.B. aus PET, erwiesen, welche einlagig oder mehrlagig ausgeführt sein können. Die Lichtauskopplung 42 gemäß eines Lambertschen Strahlers wird so weiter unterstützt beziehungsweise umgesetzt. Eine aktive Länge 40.2 des Diffusor-Elementes 40 ergibt sich dann als Abstand zwischen der Hülse 48 und der Reflektorfläche 47 und kann sich beispielsweise bis über die gesamte Länge des Diffusor-Elements 40 oder über eine aktive Länge 40.2 des Diffusor-Elements 40 erstrecken.

Zwischen Diffusor-Grundkörper 43 und der Hülle 49 aus beispielsweise Glas oder Kunststoff kann vorgesehen sein, dass zur Unterdrückung von etwaigen Oberflächenunregelmäßigkeiten, wie beispielsweise Schmutz, Rauhigkeit oder ähnlichem auf dem Diffusor-Grundkörper 43, welche das Abstrahlverhalten ungünstig beeinflussen, eine Immersionsschicht zwischen Hülle 49 und Diffusor-Grundkörper 43 eingebracht ist. Dabei ist auf einen an das Glassystem angepassten Brechungsindex einerseits, auf eine hohe Transparenz sowie auf eine ausreichend hohe Viskosität im Hinblick einer guten Applizierbarkeit zu achten. Als geeignet hat sich bspw. Glyzerin oder Silikone (Öle oder Kleber) als Immersionsschicht herausgestellt.

Zur Vermeidung von Störreflexen kann zusätzlich vorgesehen sein, dass die Reflektorfläche 47 mit einer, auf einer kurzen Länge seitlich den Diffusor-Grundkörper 43 an dessen Umfangsfläche umgreifenden Hülle oder Kappe 47.2 abgedeckt ist oder von dieser gebildet wird. In diesem Fall entspricht die aktive Länge 40.2 dem Abstand zwischen der Hülse 48 und dieser Schutzkappe. Sind die Hülse 48 beziehungsweise die Kappe 47.2 aus Metall gefertigt, kann damit eine Radio-Marker-Funktion erzielt werden, was die aktive Länge 40.2 beziehungsweise die Position des Diffusor-Elementes 40 im Röntgenbild erkennbar macht. Der gesamte Durchmesser 40.1 des Diffusor-Elementes 40 beträgt für PDT-Anwendungen typischerweise zwischen 0,8 und 1,2 mm. Üblich sind Durchmesser 40.1 von knapp 1 mm. Entscheidend ist hier der Durchmesser der Kanüle, durch die die Diffusor-Elemente 40 dem Patienten appliziert werden.

Die Befestigung von Diffusor-Grundkörper 43 und Lichtleiter 30 erfolgt innerhalb der Verbindungszone 44 durch beispielsweise einen Spleiß- oder Verklebe-Prozess mit einem brechwertangepassten hochtransparenten Kleber. Beim Spleißen wird mittels einer Corona-Entladung und/oder mittels eines Lasers, üblicherweise mit einem CO₂-Laser, der Lichtleiter 30 und der Diffusor-Grundkörper 43 anbeziehungsweise aufgeschmolzen und zusammengeführt. Je nach Material welches für den Diffusor-Grundkörper 43 und dem Lichtleiter 30 verwendet wird, kann es erforderlich sein, dass zur Anpassung derer Wärmeausdehnungskoeffizienten ein Zwischenmedium 45 verwendet wird. Dieses kann beispielsweise bei einer Glas-/Quarz-Verschmelzung ein Lot- oder Übergangsglas oder ein optischer Kleber oder Kitt sein. Denkbar und vorteilhaft zu verwirklichen ist auch eine mechanische Verpressung in Form einer Muffe, wobei lediglich der Übergang mit einem optischen Kit zur Vermeidung von Reflektionsverlusten gefüllt ist. Ebenso kann ein in der Verbindungszone 44 zwischen proximalem Ende des Diffusor-Grundkörper 46 und dem distalen Ende des Lichtleiters 30 angeordnetes optisches Element eingebeziehungsweise verbunden werden

Der Diffusor-Grundkörper 43 umfasst eine Matrix 43.4, in die die Streuelemente 43.6 in bevorzugter Anordnung parallel zur Längsachse 43.2, wie auch in der Ausführungsform der Fig. 2 gezeigt, über die gesamte Länge des Diffusor-Grundkörpers 43 eingebettet sind. Die axiale Ausdehnung einzelner Streuelemente kann dabei kleiner als die gesamte Länge des Diffusor-Grundkörpers 43 sein. Dabei können die Streuelemente 43.6 über den Durchmesser 43.1 des Diffusor-Grundkörpers 43 mehr oder weniger statistisch gleichmäßig verteilt angeordnet sind, das heißt, dass eine Mehrzahl von Streuelementen 43.6 vorhanden ist, die um die Längsachse angeordnet sind; bevorzugt sind die Streuelemente in einer regelmäßigen Struktur um die Längsachse angeordnet.

In einem Ausführungsbeispiel weist der Diffusor-Grundkörper 43 jeweils eine feste Umhüllung 43.3 und eine Matrix 43.4 auf, in die die Streuelemente 43.6 in bevorzugter Anordnung parallel zur Längsachse 43.2 über die gesamte Länge des Diffusor-Grundkörpers 43 eingebettet sind.

Fig. 6a zeigt eine Anordnung, bei der über den Durchmesser 43.1 des Diffusor-Grundkörpers 43 eine Mehrzahl Streuelemente 43.6 mehr oder weniger statistisch gleichmäßig verteilt angeordnet sind, das heißt, dass eine Mehrzahl von Streuelementen 43.6 vorhanden ist, die um die Längsachse angeordnet sind; bevorzugt sind die Streuelemente in einer regelmäßigen Struktur um die Längsachse angeordnet.

Fig. 6b zeigt eine Anordnung, bei der einzelne Streuelemente 43.6 eine insbesondere ringförmige Anordnung ausbilden, das heißt dass eine Mehrzahl von Streuelementen vorhanden ist, die um die Längsachse, bevorzugt kreisförmig angeordnet sind.

In einem weiteren Ausführungsbeispiel ist nur ein Streuelement 43.6 in Form eines Rohres oder Rohrabschnittes in die Matrix 43.4 eingebettet, das heißt, dass das zumindest eine Streuelement (43.6) rohrförmig um und insbesondere koaxial zur Längsachse angeordnet ist. Vorteilhaft bei dieser Anordnung ist eine besonders kostengünstige und reproduzierbare Herstellung der Vorform des Diffusor-Grundkörpers 43, da hier der Herstellprozess erheblich vereinfacht werden kann.

Grundsätzlich sind auch andere Geometrien für das zumindest eine Streuelement 43.6 und/oder die Anordnung einer Mehrzahl von Streuelementen 43.6, wie beispielsweise sechseckig, quadratisch, dreieckig denkbar und vorteilhaft realisierbar.

Alternativ kann vorgesehen sein, dass die Streuelemente 43.6 mehr oder weniger gleichverteilt in der Matrix 43.4 angeordnet sind, dabei aber eine Kernzone 43.7 um die Längsachse 43.2 des Diffusor-Grundkörpers 43 freilassen, das heißt, dass die Anzahl der Streuelemente 43.6 je Flächeneinheit der Querschnittsfläche des Diffusor-Grundkörpers 43 außerhalb einer Kernzone 43.7 entlang der Längsachse grösser als die je Flächeneinheit in der Kernzone 43.7 ist.

Diese Anordnung hat den Vorteil, dass das Laser-Licht, welches üblicherweise nur eine kleine numerische Apertur (NA, typ. < 0,3) aufweist, zunächst nach der Einkopplung in den Diffusor-Grundkörper 43 wenig an den Streuelementen 43.6 im Außenbereich um die Kernzone 43.7 gestreut wird, und erst nach einiger Entfernung von der Einkoppelfläche 46 verstärkt gestreut wird, wenn die einzelnen Strahlen die Streuelemente 43.6 im Randbereich erreichen. Damit kann eine Intensitätsabsenkung des seitlich abgestrahlten Lichtes direkt nach der Einkoppelfläche 46 und damit eine Homogenisierung des Intensitätsverlaufs entlang des Diffusors erreicht werden.

Bei einer konstanten Konzentration von Streuelementen entlang der Längsachse des Diffusor-Grundkörpers weist der Intensitätsverlauf einen typischerweise exponentiellen Abfall mit I₍ₗ₎ = I₀ × e ^{-l/k} auf. Dabei hat sich als günstiger Wert für k herausgestellt, wenn k in etwa der Länge des Diffusor-Grundkörpers (im konkreten Beispiel 40 mm) entspricht. Daraus ergibt sich in etwa ein 1/e-Abfall der im Betriebszustand seitlich abgestrahlten Intensität der Strahlung entlang des Diffusor-Grundkörpers, was durch die weiteren Maßnahmen derart korrigiert werden kann, dass die o.g. Homogenitätsanforderungen insbesondere für PDT-Anwendungen erfüllt werden können. In einem bevorzugten Ausführungsbeispiel wurde mit 21 Streuelementen mit jeweils einen Durchmesser von 0,3 mm als Ausgangsmaterial für die Vorform und einem Matrix-Durchmesser von etwa 600 µm (Ausgangsgeometrie Vorform 34 mm Durchmesser) ein k-Wert von 42 mm ermittelt.

In einer besonders bevorzugten Ausführungsform der Erfindung weist der Diffusor-Grundkörper 43 ferner eine feste Umhüllung 43.3 auf, welche die Matrix 43.4 zumindest abschnittsweise an der Mantelfläche umschließt. Im abgebildeten Ausführungsbeispiel der Fig. 2 ist die Mantelfläche der Matrix 43.4 vollständig von der festen Umhüllung 43.3 umschlossen, wobei auch ein zumindest abschnittsweises Umschließen bereits ausreichen kann.

Die feste Umhüllung 43.3 ist von Vorteil mit einem mehrteiligen oder mehrschichtigen Aufbau ausgebildet, umfassend zumindest zwei Hüllrohre oder Schichten, bevorzugt zumindest drei Hüllrohre (43.3.1, 43.3.2, 43.3.3) und/oder Schichten.

Zur Homogenisierung des Intensitätsverlaufs aber auch insbesondere zur Verhinderung von ungewollter Zurückleiten des Lichtes in die Zuleitungsfaser, was insbesondere die Stabilität einer als Laser-Lichtquelle ausgebildeten Lichtquelle 10 beeinträchtigen kann bis hin zu Störungen in deren Regelung oder sogar zum Abschalten der Laser-Lichtquelle, oder zu ungewollter Erwärmung der Steckverbindung führen kann, sind erfindungsgemäß weitere Maßnahmen zur Aufweitung der numerischen Apertur NA des an der Reflektorfläche 47 rückreflektierten Lichtes 42.1 denkbar.

Dies kann in vorteilhafter Weise mit einem zumindest bereichsweisen Anschliff der distalen Endfläche bzw. der Reflektorfläche 47 unter einem Winkel ungleich von 90° zur Längsachse 43.2 des Diffusor-Grundkörpers 43 realisiert werden.

Weitere Ausführungsformen der Reflektorfläche 47 zeigen schematisch die Figuren 8a und 8b. Demnach kann zur Homogenisierung des Intensitätsverlaufs vorgesehen sein, dass die Reflektorfläche 47 konkav (Fig. 8a) oder konvex (Fig. 8b) ausgeformt ausgebildet ist. Damit kann erreicht werden, dass reflektierte Strahlen mit nahezu parallelem Verlauf zur Längsachse 43.2 unter steilerem Winkel zur Längsachse 43.2 zurückreflektiert und damit an den Streuelementen 43.6 häufiger gestreut werden, so dass die Auskoppeleffizienz am distalen Ende des Diffusor-Elementes 40 erhöht wird.

Zusätzlich kann damit ein Strahlungsanteil, welcher insbesondere in den Lichtleiter 30 zurückreflektiert wird, weitgehend reduziert werden. Weitere, insbesondere auch kostengünstig realisierbare Maßnahmen können ein Anschliff des Diffusor-Grundkörpers 43 unter einem Winkel ungleich 90° zur Längsachse 43.2 sein. Ebenso wirksam kann ein facetten-artiger Anschliff sein bei dem die einzelnen Flächen unterschiedliche Winkel, überwiegend jeweils ungleich 90° zur Längsachse 43.2 des Diffusor-Grundkörpers 43 ausbilden. Die Winkelabweichung zu 90° liegt üblicherweise unter 5°.

Die Reflektorfläche 47 am distalen Ende des Diffusor-Grundkörpers 43 kann auch als hohler und/oder transparenter Körper 47.1 mit einer in den Hohlraum und/oder in den transparenten Körper hinein reflektierenden Hülle 47.2 ausgebildet sein, wie dies Fig. 8c schematisch zeigt. Die Hülle 47.2 kann als bevorzugt gerichtet oder diffus reflektierende Beschichtung und/oder Kappe ausgeführt sein. Diese können auch ohne Hohlraum direkt mit dem Diffusor-Grundkörpers 43 abschließen und diesen in beiden Fällen über eine kurze Länge am distalen Enden zumindest teil- oder abschnittsweise an dessen Umfang radial umfassen.

Demnach kann die Reflektorfläche 47 konkav oder konvex und/oder als direkt oder beabstandet, einen Hohlraum zwischen Reflektorfläche 47 und distalem Ende des Diffusor-Grundkörpers 43 bildend, an den Diffusor-Grundkörper 43 anschließender, Körper 47.1 und/ oder Hülle 47.2 als einseitig geschlossener Hohlkörper ausgebildet sein.

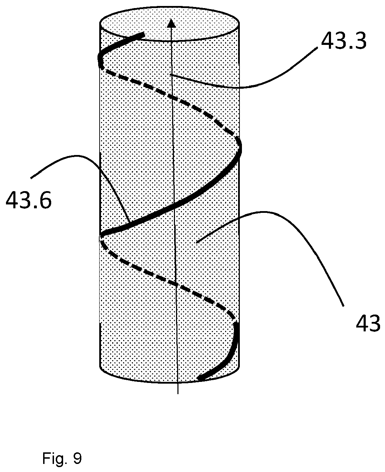

Fig. 9 zeigt schematisch beispielhaft nur ein filamentartiges Streuelement 43.6 des Diffusor-Grundkörpers 43 in einer Helix-förmigen Anordnung um die Längsachse 43.2 des Diffusor-Grundkörpers 43. Eine solche Helix-Struktur kann erzielt werden, wenn zusätzlich beim Ausziehen der Anordnung in einer Ziehanlage die Vorform um die Längsachse der Vorform verdreht werden, um damit diese Helix-förmige Abordnung der Streuzentren 43.6 um die Längsachse 43.2 zu erzeugen.

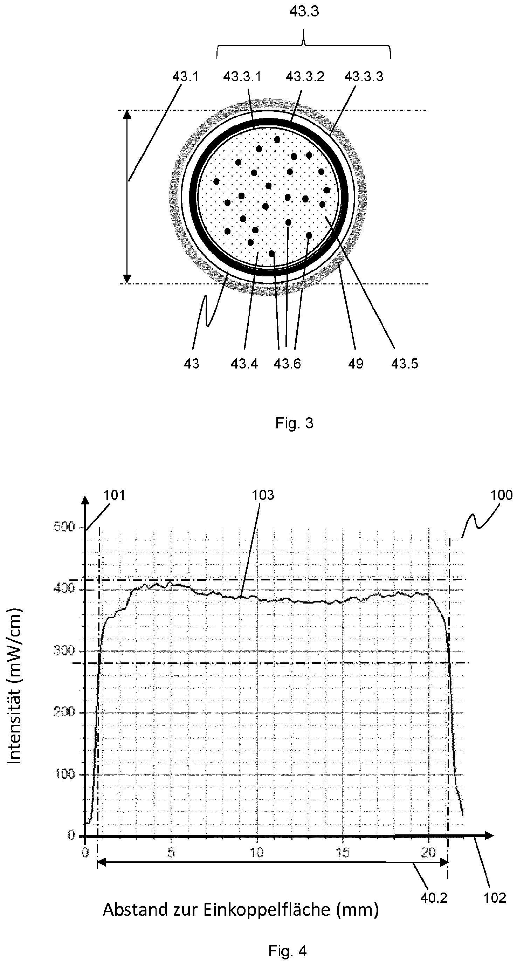

Fig. 3 zeigt in einem schematisch dargestellten Querschnitt einen erfindungsgemäßen Diffusor-Grundkörper 43 mit seinem Durchmesser 43.1 sowie einer die Matrix 43.4 umgebenden festen Umhüllung 43.3, bei dem diese die Matrix 43.4 mit den Streuelementen 43.6 umgebende feste Umhüllung 43.3 aus mehreren koaxialen Hüllrohren 43.3.1, 43.3.2, 43.3.3 aufgebaut ist, welche unterschiedliche optische Eigenschaften aufweisen. Die Matrix 43.4 ist dabei aus einzelnen Matrix-Elementen 43.5 aufgebaut.

Im Sinne der Erfindung sind aber auch bereits Ausführungsformen mit zwei koaxial zueinander angeordneten Hüllrohren 43.3.1, 43.3.2 möglich und denkbar.

Gemäß des in Fig. 3 abgebildeten, besonders bevorzugten Ausführungsbeispiels der Erfindung ist das erste Hüllrohr 43.3.1, welches die Matrix 43.4 umgibt, aus einem transparenten Material, wobei der Brechungsindex niedriger ist als der Brechungsindex der Matrix 43.4. Dies wird auch als optisches Cladding bezeichnet und sorgt dafür, dass das eingekoppelte Licht gemäß der Lichteinkopplung 41 zunächst in der Matrix 43.4 geführt und damit mit den Streuelementen 43.6 wechselwirken kann, so dass her bereits eine hohe Streueffizienz erzielt werden kann.

Das zweite Hüllrohr 43.3.2 ist in dem Ausführungsbeispiel als streuendes, d.h. transluzentes Rohr ausgebildet, indem weitere Streuelementen eingelagert sind. Hiermit können insbesondere sogenannte Mantelmoden, welche an der Reflektorfläche 47 zurück in den Mantel bzw. in das erste Hüllrohr 43.3.1 eingekoppelt werden, aus dem Diffusor-Grundkörper 43 ausgekoppelt werden, was die Abstrahlintensität der Abstrahlung 42 zusätzlich unterstützt. Das zweite Hüllrohr 43.3.2 kann auch ersatzweise als eine ringförmige Anordnung von einzelnen stark streuenden Weißglasstäbchen ausgeführt sein.

Dabei muss das zweite streuende Hüllrohr 43.3.2 optisch an das erste Hüllrohr 43.3.1 angekoppelt sein und der Brechungsindex des zweiten Hüllrohrs 43.3.2 höher sein als der Brechungsindex des ersten Hüllrohrs 43.3.1. Damit kann aufgrund der Mehrfachstreuung im zweiten Hüllrohr 43.3.2 eine nahezu isotrope Abstrahlung erreicht werden. Zudem wird damit eine zusätzliche Homogenisierung erreicht.

Das äußere dritte Hüllrohr 43.3.3 dient als mechanische Stabilisierung insbesondere für das zweite Hüllrohr 43.3.2 und erlaubt es damit, durch den Ziehprozess einen kompakten und robusten Diffusor-Grundkörper 43 auszubilden. Zusätzlich kann wie bereits vorstehend zu Fig. 2 beschrieben eine Hülle 49 aus einem dünnwandigen Polymermaterial, welches klar transparent, leicht transluzent und/oder auch farblich dotiert ausgeführt sein kann, den Diffusor-Grundkörper 43 sowie einen Teil des Lichtleiters 30 umschließen.

In bevorzugter Ausführungsform besteht die Matrix 43.4 aus einem optisch hochtransparenten Glas, wie es z.B. das optische Glas SCHOTT N BK-7 der Fa. Schott AG darstellt. Die Streuelemente 43.6 können z.B. aus einem Weißglas bestehen.

Als erstes Hüllrohr 43.3.1 und als drittes Hüllrohr 43.3.3 hat sich ein hoch transparentes borosilikathaltiges Glas mit der Bezeichnung SCHOTT FIOLAX^{®} 8412 der Fa. Schott AG als besonders geeignet erwiesen. Dieses weist in einem Wellenlängenbereich von 250 bis 2.000 nm Wellenlänge eine Transmission von etwa 92 % auf und gilt damit als hoch transparent im Sinne der Erfindung.

Das zweite Hüllrohr 43.3.2 besteht in einer bevorzugten Ausführungsform aus einem Weißglasrohr und kann dabei ähnlich oder gleich aufgebaut sein, wie dies bei den Streuelementen 43.6 in der Matrix 43.4 der Fall ist, wenn dafür Weißglas eingesetzt wird.

Dieser Aufbau hat den Vorteil, dass all diese Komponenten sehr gut miteinander verschmelzbar sind und damit erlauben, einen kompakten Diffusor-Grundkörper 43 auszubilden, der keine Hohlräume z.B. in Form von Luftspalten oder Luftblasen enthält.

Typische Schichtdicken für die Hüllrohre 43.3.1, 43.3.2, 43.3.3 liegen bei einem Durchmesser 43.1 des Diffuser-Grundkörpers 43 von 500 µm in einem Bereich von 5 µm bis 50 µm, bevorzugt von 7 bis 40 µm, besonders bevorzugt bei etwa 10 µm bis 30 µm oder bei 10 µm.

Andere Aufbauten der festen Umhüllung 43 sind möglich und denkbar. So kann die feste Umhüllung 43 selbstverständlich auch aus einer Kombination von Hüllrohren und/oder Schichten oder beispielsweise mehr als drei Hüllrohren oder Schichten aufgebaut sein, falls eine weitere Funktionalisierung gewünscht ist. Es ist somit auch möglich, weitere Hüllrohre vorzusehen, welche dann das dritte Hüllrohr zumindest abschnittsweise umschließen.

Denkbar sind auch Aufbauten mit nur einem ersten Hüllrohr 43.3.1 und einem zweiten streuenden Hüllrohr 43.3.2. Bei einer Ausführungsform der Erfindung mit nur zwei Hüllrohren 43.3.1, 43.3.2 können das erste Hüllrohr 43.3.1 hoch transparent und das zweite Hüllrohr 43.3.2 als Weißglasrohr ausgebildet sein wie vorstehend erläutert. In diesem Fall kann auch bereits das zweite Hüllrohr 43.3.2 für die mechanische Stabilisierung sorgen.

Die Fig. 7a und 7b zeigen schematisch zwei weitere Ausführungsbeispiele für den Aufbau der Matrix 43.4 im Diffusor-Grundkörper 43 in einem Querschnitt senkrecht zu der Längsachse des Diffusor-Grundkörpers 43.

Fig. 7a zeigt beispielhaft ein Streuelement 43.6 welches in der Vorform als dünnes Stäbchen zwischen den Matrix-Elementen 43.5 in Form von Einzelstäben eingelagert ist. Im gezeigten Beispiel füllt das Streuelement 43.6 die Zwischenräume (Zwickel) von drei Einzelstäben als Matrix-Elemente 43.5 aus. Im gezeigten Beispiel wurden für die Herstellung der Vorform Einzelglasstäbe von 2 mm Durchmesser als Matrix-Elemente 43.5 verwendet. Die Streuelemente sind aus 0,3 mm dicken Weißglasstäben gebildet. Nach dem thermischen Ziehprozess, d.h. nach dem Herunterziehen auf den Durchmesser 43.1 des Diffusor-Grundkörpers 43 ist das Streuelement 43.6 an- oder aufgeschmolzen und weist einen dreieckigen, beispielsweise insbesondere hyperbolisch dreieckigen Querschnitt auf.

Fig. 7b zeigt eine alternative Anordnung, bei der die Durchmesser der Streuelemente 43.6 gleich groß oder kleiner sind als die Durchmesser der als Einzelstäbe ausgebildeten Matrix-Elemente 43.5. Hier liegen die typischen Durchmesser vor dem Ziehprozess in der entsprechend zusammengesetzten Vorform im Bereich von 0,5 bis 1 mm für die Streuelemente 43.6 als beispielsweise Weißglasstäbchen und die Matrix-Elemente 43.5. Nach dem thermischen Ziehprozess, das heißt nach dem Herunterziehen auf den Durchmesser 43.1 des Diffusor-Grundkörpers 43 ist das Streuelement 43.6 an- oder aufgeschmolzen und weist einen sechseckigen, beispielsweise insbesondere hyperbolisch sechseckigen Querschnitt auf.

Die Anordnung der Streustäbe in den Zwickeln der Vorform ermöglicht dabei bei gegebener Größe der Lichtleitstäbe und gegebenem Querschnittsanteil eine höhere Anzahl der Streukörper und damit eine bessere Homogenität zu erzielen. Die Matrix-Elemente 43.5 und die Streuelemente 43.6 können nach dem Ziehprozess als Diffusor-Grundkörper 43 einen runden, sechseckigen, quadratischen oder dreieckigen Querschnitt, insbesondere dessen hyperbolische Varianten, aufweisen.

Die in dem Diffusor-Grundkörper 43 eingelagerten Streuelemente 43.6, welche in der Vorform als dünne Stäbchen zwischen den Matrix-Elementen 43.5 der Matrix 43.4 in Form von Einzelstäben eingelagert sind, füllen die Zwischenräume (Zwickel) von drei Einzelstäben als Matrix-Elemente 43.5 aus. Typischerweise werden für die Herstellung der Vorform Einzelglasstäbe von 2 mm Durchmesser als Matrix-Elemente 43.5 verwendet. Die Streuelemente sind aus 0,3 mm dicken Weißglasstäben gebildet. Nach dem thermischen Ziehprozess, d.h. nach dem Herunterziehen auf den Durchmesser 43.1 des Diffusor-Grundkörpers 43 ist das Streuelement 43.6 an- oder aufgeschmolzen und weist einen dreieckigen, beispielsweise insbesondere hyperbolisch dreieckigen Querschnitt auf. Denkbar sind auch alternative Anordnungen, bei der die Durchmesser der Streuelemente 43.6 gleich groß oder kleiner sind als die Durchmesser der als Einzelstäbe ausgebildeten Matrix-Elemente 43.5. Hier liegen die typischen Durchmesser vor dem Ziehprozess in der entsprechend zusammengesetzten Vorform im Bereich von 0,5 bis 1 mm für die Streuelemente 43.6 als beispielsweise Weißglasstäbchen und die Matrix-Elemente 43.5. Nach dem thermischen Ziehprozess, das heißt nach dem Herunterziehen auf den Durchmesser 43.1 des Diffusor-Grundkörpers 43 ist das Streuelement 43.6 an- oder aufgeschmolzen und weist einen sechseckigen, beispielsweise insbesondere hyperbolisch sechseckigen Querschnitt auf.

Die Anordnung der Streustäbe in den Zwickeln der Vorform ermöglicht dabei bei gegebener Größe der Lichtleitstäbe und gegebenem Querschnittsanteil, eine höhere Anzahl der Streukörper und damit eine bessere Homogenität zu erzielen. Die Matrix-Elemente 43.5 und die Streuelemente 43.6 können nach dem Ziehprozess als Diffusor-Grundkörper 43 einen runden, sechseckigen, quadratischen oder dreieckigen Querschnitt, insbesondere dessen hyperbolische Varianten, aufweisen.

Eine Ausführungsform der als Weißglasstäbe ausgebildeten Streuelemente 43.6 oder des Weißglasrohrs bzw. des streuenden Hüllrohrs sieht vor, dass in diesen Streuzentren durch Streupartikel gebildet werden, wobei die Konzentrationen der Streupartikel im Streubereich von 10 ppm bis 1000 ppm und bevorzugt von 20 ppm bis 100 ppm beträgt.

Die Effizienz der Auskopplung aus dem Streubereich, somit dem Volumen des Weißglases der Streustäbe oder des Weißglasrohrs ist neben der streuenden Eigenschaft der Streupartikel als intrinsischem Parameter auch von der Konzentration der Streupartikel im Streubereich selbst abhängig.

Die Konzentrationsangabe in ppm bezieht sich hierbei auf den Anteil der Streupartikel im Verhältnis zu den Masseanteilen der Bestandteile des Weißglases, in welchem die Streupartikel eingelagert sind.

Dienen inhomogene Bereiche des Weißglases als Streuzentren, ergibt sich eine alternative Ausführungsform, in der die inhomogenen Bereiche bevorzugt durch Phasentrennung und/oder Entmischung der Glaskomponenten des Glases gebildet werden, in welches sie eingelagert sind.

Die durch inhomogene Bereiche gebildeten Streuzentren weisen bevorzugt einen Durchmesser von 10 nm bis 1000 nm auf, besonders bevorzugt von 100 nm bis 800 nm.

Besonders bevorzugt sind diese Streuzentren kugelförmig. Für nicht kugelförmige Streuzentren wird als Durchmesser ihre maximale Ausdehnung verstanden.

Das Glas, welche vorliegend als Weißglas bezeichnet wird, in dem die inhomogenen Bereiche als Streuzentren eingelagert sind, kann bevorzugt aus einem As- und Pbhaltigen Silikatglas bestehen. Die Streuzentren weisen in diesem Fall bevorzugt einen gegenüber der umgebenden Glasmatrix erhöhten Gehalt an Pb und/oder As auf.

Alternativ kann das Glas beziehungsweise Weißglas, in welches die inhomogenen Bereiche als Streuzentren eingelagert sind, aus einem Fluor-haltigen Ca-Zn-Silikatglas bestehen. Dann weisen die Streuzentren einen gegenüber der umgebenden Glasmatrix bevorzugt einen erhöhten Gehalt an Fluor auf.

In Fig. 4 ist in einem Verlaufsdiagramm 100 ein gemessener Intensitätsverlauf 103 am Beispiel eines Zylinder-Diffusors mit einer aktiven Länge 40.2 von ca. 20 mm dargestellt. Dargestellt ist die Intensität 101, hier gemessen als "radiant Excitance" (spezifische Ausstrahlung) in mW/cm Diffusorlänge in Abhängigkeit vom Abstand zur Einkoppelfläche 102.

Der Intensitätsverlauf 103 zeigt einen insgesamt relativ konstanten Verlauf gegenüber einem sonst typischerweise leicht exponentiellen Abfall der Intensität 101, wie er sich als Lösung einer Differentialgleichung für einen über die Länge homogenen Streuverlauf, d.h. ein konstantes Verhältnis von eingestrahlter Strahlung zu gestreuter Strahlung in einem Längenabschnitt liegt vor, ergibt.

Durch Anbringen einer Reflektorfläche 47 am distalen Ende des Diffusor-Grundkörpers 43 (vergl. Fig. 2) kann ein Teil der Strahlung wieder zurückreflektiert werden, welche dann insbesondere im Bereich vor der Reflektorfläche 47 zusätzliche Streubeiträge liefert. Mathematisch bedeutet dies eine Addition von zwei Exponential-Funktionen.

Durch den besonderen Aufbau der festen Umhüllung 43.3 mit zumindest zwei oder sogar drei Hüllrohren sowie den zusätzlichen Maßnahmen zur Aufweitung der NA des rückreflektierten Lichtes zur Erhöhung der Streueffizienz kann ein nahezu homogener Intensitätsverlauf mit Abweichungen von < ± 10 % vom Mittelwert über die aktive Diffusorlänge 40.2 erzielt werden. Zudem kann die Gesamt-Effizienz der gewünschten seitlichen Abstrahlung auf > 85 %, typ. > 90 % gesteigert werden. Die aktive Diffusorlänge entspricht dabei typischerweise der Länge des Diffusor-Grundkörpers.

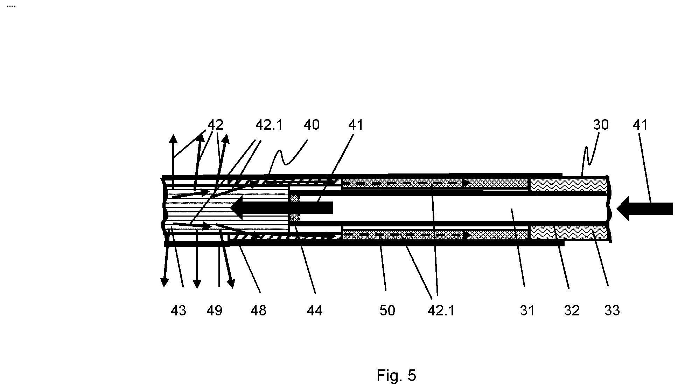

Fig. 5 zeigt in einer schematischen Darstellung ausschnittsweise einen Aufbau zur Vermeidung von rückstreuenden Licht in den äußeren Mantel des Lichtleiters. Dieses Phänomen kann einerseits ein Aufleuchten des Lichtleiters 30 bewirken, was noch eher bei geringen Leistungen als nur kosmetischer Effekt behandelt werden kann.

Allerdings kann, insbesondere bei höherer Laserleistung, beispielsweise größer 2 W, und auch höherem rückgestreuten Lichtanteil daraus auch eine unzulässige Erwärmung resultieren, insbesondere in Bereichen, wo dieses rückreflektierte Licht auf Komponenten trifft, die dieses Licht in einem kleinen Bereich absorbieren. Hier hat sich insbesondere rückgestreutes Licht 42.1, welches über die transparente Hülse 48 in Richtung Laser-Lichtquelle geleitet wird und dann auf den äußeren Mantel 33 des Lichtleiters 30 trifft als störend erwiesen. Hier kommt es, abhängig von der Intensität der verwendeten Laserleistung beziehungsweise der Lichtstrahlung, zum einen zu einem Aufleuchten der Stirnfläche des äußeren Mantels 33, was recht intensiv sein kann, und was dann auch zu einer starken Erwärmung führen kann.

Abhilfe kann hier eine transluzente bzw. teilabsorbierende Hülle 50 schaffen, die das rückgestreute Licht 42.1 aus der Hülse teilweise streut und somit die Intensität im Auftreffbereich auf die äußeren Mantel 33 des Lichtleiters deutlich reduziert. Das rückgestreute Licht 42.1 kann durch Teilabsorption und/oder Mehrfachstreuung aus der teilabsorbierenden Hülle 50 heraus minimiert bzw. über eine größere Fläche verteilt werden, so dass dieses rückgestreute Licht i) nicht oder kaum mehr wahrnehmbar ist und ii) die Leistungsdichte im Hinblick auf eine Erwärmung deutlich reduziert ist.

Die erfindungsgemäße teilabsorbierende Hülle 50 füllt daher idealerweise den Zwischenraum zwischen der Hülse 48 und dem äußeren Mantel 33 des Lichtleiters 30 aus, wie dies in Fig. 5 schematisch dargestellt ist. Diese teilabsorbierende Hülle 50 kann als separates Element in Form eines transluzenten Schlauchabschnitts oder eines Schrumpfschlauches und/oder als Re-Coating-Material, in dem streuende Partikel verteilt sind, realisiert werden. Da ein Re-Coating der Faser im Bereich der Verbindungszone 44 bzw. bis zum Beginn des äußeren Mantels 33 zur Wahrung des Festigkeitsniveaus erforderlich ist, kann letzteres in einem Arbeitsschritt realisiert werden. Typische Re-Coating-Materialien bestehen aus einem Acrylat oder einem Epoxi-Material und dienen zur nachträglichen Versiegelung der Oberfläche des Lichtleiters 30, was insbesondere die mechanische Stabilität erhöht.

Diese Maßnahme stellt eine weitere Ergänzung der zuvor beschriebenen Maßnahmen zur Vermeidung einer zu großen rückgestreuten Lichtintensität dar und trägt somit ebenfalls zur Optimierung des Diffusor-Systems gemäß der Aufgabenstellung bei.

**Bezugszeichenliste:**

| | |
|---|---|
| 1 | Beleuchtungssystem |
| 10 | Lichtquelle |
| 20 | Stecker |
| 30 | Lichtleiter |
| 31 | Kern |
| 31.1 | Kerndurchmesser oder Faserbündeldurchmesser |
| 32 | Mantel |
| 33 | Äußerer Mantel |
| 40 | Diffusor-Element |
| 40.1 | Durchmesser |
| 40.2 | aktive Länge |
| 41 | Lichteinkopplung |
| 42 | Lichtauskopplung |
| 42.1 | Rückgestreutes Licht |
| 43 | Diffusor-Grundkörper |
| 43.1 | Durchmesser |
| 43.2 | Längsachse |
| 43.3 | feste Umhüllung |
| 43.3.1 | 1. Hüllrohr |
| 43.3.2 | 2. Hüllrohr |
| 43.3.3 | 3. Hüllrohr |
| 43.4 | Matrix |
| 43.5 | Matrix-Element |
| 43.6 | Streuelement |
| 43.7 | Kernzone |
| 43.8 | Längsachse des Streuelements, insbesondere Weißglasstäbchens |
| 43.9 | Weißglasstäbchen |
| 43.10 | Winkel |
| 44 | Verbindungs zone |
| 45 | Zwischenmedium |
| 46 | Einkoppelfläche |
| 47 | Reflektorfläche |
| 47.1 | Körper |
| 47.2 | Reflektierende Hülle / Kappe |
| 48 | Hülse |
| 49 | Hülle |
| 50 | Teilabsorbierende Hülle |
| 60 | Blockierelement |
| 70 | Gewebe |
| 80 | Tumorgewebe |
| 100 | Verlaufsdiagramm |
| 101 | Intensität |
| 102 | Abstand zur Einkoppelfläche |
| 103 | Intensitätsverlauf |

## Patentansprüche

1. Beleuchtungssystem (1) insbesondere für ein medizintechnisches Therapie- und/oder Diagnosesystem, umfassend wenigstens eine Lichtquelle (10), und einen Lichtleiter (30), der an einem proximalen Ende an die wenigstens eine Lichtquelle (10) anschließbar und/oder angeschlossen ist, und welches am distalen Ende des Lichtleiters (30) ein Diffusor-Element (40) mit einer Längsachse aufweist, die senkrecht zu einer Einkoppelfläche des Lichtleiters (30) in das Diffusor-Element (40) verläuft,
wobei das Diffusor-Element (40) im Betriebszustand Licht über seine aktive Länge (40.2) seitlich zur Längsachse abstrahlt,
wobei das Diffusor-Element (40) mindestens einen Diffusor-Grundkörper (43) aufweist, welcher eine Matrix (43.4) mit mindestens einem Streuelement (43.6) umfasst, welche von einer festen Umhüllung (43.3) zumindest abschnittsweise an seiner Mantelfläche umschlossen ist, und
wobei die feste Umhüllung (43.3) mit einem mehrteiligen oder mehrschichtigen Aufbau ausgebildet ist, umfassend zumindest zwei Hüllrohre oder Schichten, bevorzugt zumindest drei Hüllrohre (43.3.1 ... 43.3.3) und/oder Schichten.

2. Beleuchtungssystem (1) nach Anspruch 1, umfassend ferner zumindest eines der folgenden Merkmale:
das zumindest eine Streuelement (43.6) ist entlang der Längsachse (43.2) des Diffusor-Grundkörpers (43) im Wesentlichen zu dieser parallel ausgerichtet, in einem Winkel zur Längsachse (43.2), oder spiralförmig, insbesondere in Form einer Spirale mit konstanter Steigung und/oder Helix-förmig um die Längsachse (43.2) angeordnet,
am distalen Ende des Diffusor-Grundkörpers (43) und/oder dem Übergangsbereich zwischen Lichtleiter (30) und Diffusor-Grundkörper (43) und/oder dem Diffusor-Grundkörper (43) sind, diesen zumindest teilweise oder abschnittsweise umschließend, Einrichtungen zur Homogenisierung der Abstrahlungsintensität entlang der Längsachse (43.2) des Diffusor-Grundkörpers (43) vorgesehen,
der Diffusor-Grundkörper (43) weist an seinem distalen Ende eine Reflektorfläche (47) auf, mit der das Diffusor-Grundkörper (43) durchlaufende Licht zumindest teilweise wieder zurück reflektierbar ist,
das Beleuchtungssystem weist im Betriebszustand eine Intensitätsverteilung der seitlichen Abstrahlung auf, die um höchstens ± 50 %, bevorzugt höchstens ± 30 % und meist bevorzugt höchstens als ± 5 % von der mittleren seitlichen Abstrahlungsintensität abweicht.

3. Beleuchtungssystem (1) nach einem der vorstehenden Ansprüche, wobei sich zumindest zwei der Hüllrohre (43.3.1 ... 43.3.3) in zumindest einer optischen Eigenschaft voneinander unterscheiden, wobei die optische Eigenschaft vorzugsweise die Transparenz, den Brechungsindex und/oder das Material des Hüllrohrs umfasst.

4. Beleuchtungssystem (1) nach einem der vorstehenden Ansprüche, wobei ein erstes Hüllrohr (43.3.1) die Matrix (43.4) zumindest abschnittsweise oder vollständig umgibt und wobei vorzugsweise das erste Hüllrohr (43.3.1) im Wesentlichen klar transparent ausgebildet ist und/oder einen kleineren Brechungsindex aufweist als der Brechungsindex des Materials der Matrix (43.4).

5. Beleuchtungssystem (1) nach einem der vorstehenden Ansprüche, wobei ein zweites Hüllrohr (43.3.2) das erste Hüllrohr (43.3.1) zumindest abschnittsweise oder vollständig umgibt, und wobei vorzugsweise das zweites Hüllrohr (43.3.2) transluzent bzw. streuend ausgebildet ist und/oder einen größeren Brechungsindex aufweist als der Brechungsindex des ersten Hüllrohres (43.3.1).

6. Beleuchtungssystem (1) nach einem der vorstehenden Ansprüche, wobei das das erste Hüllrohr (43.3.1) ein transparentes Borosilikatglas umfasst, und/oder wobei das zweite Hüllrohr (43.3.2) ein Weißglas umfasst oder aus einer ringförmigen Anordnung aus einzelnen Weißglasstäbchen besteht.

7. Beleuchtungssystem (1) nach einem der vorstehenden Ansprüche, wobei ein drittes Hüllrohr (43.3.3) vorgesehen ist, welches das zweite Hüllrohr (43.3.2) zumindest abschnittsweise oder vollständig umgibt, und wobei vorzugsweise das dritte Hüllrohr (43.3.3) im Wesentlichen klar transparent ausgebildet ist.

8. Beleuchtungssystem (1) nach einem der vorstehenden Ansprüche, wobei zumindest ein Hüllrohr Röntgen-opakes Glas umfasst.

9. Beleuchtungssystem (1) nach einem der vorstehenden Ansprüche, wobei die Matrix (43.4) mit dem mindestens einen Streuelement (43.6) und die fest Umhüllung als geschlossener, dichter Verbund ohne Hohlräume oder Luftblasen ausgeführt ist.

10. Beleuchtungssystem (1) nach einem der vorstehenden Ansprüche, wobei das Diffusor-Element (43) mit der Matrix (43.4), dem mindestens einen Streuelement (43.6) und/oder die Hüllrohre der festen Umhüllung (43.3, 43.3.1 ... 43.3.3) aus Glas aufgebaut sind oder Glas umfassen, wobei diese Komponenten vorzugsweise zu einem kompakten, geschlossenem Körper miteinander verschmolzen sind.

11. Beleuchtungssystem (1) nach einem der vorstehenden Ansprüche, wobei die wenigstens eine Lichtquelle (10) eine Laser-Lichtquelle oder halbleiterbasierte Lichtquelle oder lichtemittierende Diode (LED), eine Laserdiode (LD), oder einen Laser umfasst.

12. Beleuchtungssystem (1) nach einem der vorhergehenden Ansprüche, wobei auf dem distalen Ende des Diffusor-Grundkörpers (43) eine Reflektorfläche (47) vorgesehen ist, die den Diffusor-Grundkörpers (43) abschließt und/oder zumindest teilweise oder abschnittsweise an dessen Umfangsfläche umfasst, wobei im Betrieb das Licht direkt und/oder diffus zurück reflektierbar ist, und
wobei die Reflektorfläche (47) vorzugsweise als aufgesputterte oder aufgedampfte dielektrische Reflektionsschicht auf dem distalen Ende des Diffusor-Grundkörpers (43) ausgebildet ist.

13. Beleuchtungssystem (1) nach vorstehendem Anspruch, wobei die Reflektorfläche (47) mehrere Schichten umfasst, wobei die Schichten vorzugsweise derart ausgewählt sind, dass ein Maximum der Reflektivität bei der Wellenlänge des Lichtes der wenigstens einen Lichtquelle (10) besteht.

14. Beleuchtungssystem (1) nach einem der beiden vorstehenden Ansprüche, wobei das Maximum der Reflektivität derart ausgewählt ist, dass ein erstes Maximum der Reflektivität bei einer Anwendungswellenlänge besteht und zumindest ein weiteres, zweites Maximum der Reflektivität bei einer weiteren Wellenlänge, wobei sich diese weitere Wellenlänge von der Anwendungswellenlänge unterscheidet, und wobei die Reflektivität des ersten Maximums und die des zumindest einen weiteren Maximums > 95 %, bevorzugt > 99 % beträgt.

15. Beleuchtungssystem (1) nach mindestens einem der vorhergehenden Ansprüche, wobei die Reflektorfläche (47) zumindest abschnittsweise einen Winkel von kleiner 90° zur Längsachse (43.2) des Diffusor-Grundkörpers (43) ausbildet und im Betrieb rückreflektiertes Licht (42.1) mit einer größeren numerischen Apertur NA reflektierbar ist als das Licht, welches auf die Reflektorfläche (47) trifft.

16. Beleuchtungssystem (1) nach mindestens einem der vorhergehenden Ansprüche, wobei im Bereich der Verbindungszone (44) zwischen Lichtleiter (30) und Diffusor-Grundkörper (43.3) eine transparente Hülse (48), vorzugsweise Glas oder Kunststoff umfassend, angeordnet ist und
wobei vorzugsweise im Bereich zwischen der Hülse (48) und einem äußeren Mantel (33) des Lichtleiters eine transluzente bzw. teilabsorbierende Hülle (50) vorgesehen ist und diese transluzente bzw. teilabsorbierende Hülle (50) aus einem Polymer, in welches Streupartikel eingelagert sind, ausgebildet ist.

17. Beleuchtungssystem (1) nach vorstehendem Anspruch, wobei die transluzente bzw. teilabsorbierende Hülle (50) ein Schlauchabschnitt, ein Schrumpfschlauchabschnitt und/oder ein Re-Coating-Polymer ist, in das Streupartikel zuvor einbringbar sind.

18. Verfahren zum Herstellen eines Diffusor-Grundkörpers, insbesondere für ein Beleuchtungssystem nach einem der vorstehenden Ansprüche, aufweisend die Verfahrensschritte:
- Bereitstellen einer Mehrzahl von Lichtleitstäben aus einem Glas mit dem Brechungsindex n₁ und/oder n₁',
- Anordnen der Mehrzahl der Lichtleitstäbe mit dem Brechungsindex n₁ und/oder n₁' und zumindest eines Streustabs aus einem Glas oder einer Glaskeramik, die Streuzentren umfasst, so dass die Längsachsen der Lichtleitstäbe und des zumindest einen Streustabs im Wesentlichen, parallel zueinander verlaufen und eine Vorform erhalten wird,
- Erwärmen der Vorform,
- Ausziehen der Vorform in einer Ziehanlage und ggf. Ablängen, um einen Diffusor-Grundkörper zu erhalten.

19. Verfahren zum Herstellen eines Diffusor-Grundkörpers nach vorstehendem Anspruch, ferner aufweisend die folgenden Schritte:
- Bereitstellen einer Matrix mit dem zumindest einem Streuelement,
- Schaffen einer Anordnung, bei welcher die Matrix mit dem zumindest einem Streuelement im Innern des ersten Hüllrohres angeordnet wird,
- Platzieren des ersten Hüllrohres in einem zweiten Hüllrohr,
- Ausziehen der Anordnung in einer Ziehanlage und ggf. Ablängen, um einen Diffusor-Grundkörper mit fester Umhüllung zu erhalten.

20. Verfahren zum Herstellen eines Diffusor-Grundkörpers nach Anspruch 19, wobei das Verfahren einen zusätzlichen Schritt aufweist, bei dem beim Ausziehen der Anordnung in einer Ziehanlage die Vorform um die Längsachse der Vorform verdreht wird und damit eine Helix-förmige Abordnung der Streuzentren (43.6) um die Längsachse (43.2) erzeugt wird.

21. Verfahren zum Herstellen eines Diffusor-Grundkörpers nach vorstehendem Anspruch, ferner aufweisend den folgenden Schritt:
- Platzieren des zweiten Hüllrohres in einem dritten Hüllrohr, bevor die Anordnung in einer Ziehanlage ausgezogen wird.

22. Verwendung des Beleuchtungssystems (1) nach einem der vorstehenden Ansprüche als Komponente einer Vorrichtung für ein medizinisches Therapieverfahren, insbesondere für eine photodynamische Therapie (PDT) oder Photo-Immuno-Therapie (PIT) zur Tumortherapie, für eine endovenöse Lasertherapie (EVLT) zur Behandlung von Krampfadern, für eine Laser- induzierte interstitielle Thermotherapie (LITT) oder für Anwendungen im Bereich der Dentalmedizin, Augenheilkunde sowie Dermatologie.

23. Verwendung des Beleuchtungssystems (1) nach einem der vorstehenden Ansprüche als Komponente einer Vorrichtung für eine photodynamische Therapie (PDT) oder Photo-Immuno-Therapie (PIT) zur Tumortherapie, wobei zumindest ein Lichtleiter (30) mit dem Diffusor-Element (40) aus anderen Diffusor-Elementen (40) abgestrahltes Licht aufnimmt und über den Lichtleiter (30) einem Detektor zur spektroskopischen Analyse und/ oder zu Dosimetriebetrachtungen weiterleitet.
